# EUROPEAN PATENT SPECIFICATION

(11) **EP 4 611 841 B1**
(45) Date of publication and mention of the grant of the patent: **16.09.2026**
(21) Application number: 23814032.1
(22) Date of filing: 30.10.2023
(51) Int. Cl.: G16H 20/17, A61M 5/142, A61M 5/315

(54) **MEDICATION DELIVERY DEVICE WITH SIGNAL FILTERING**
MEDIKAMENTENABGABEVORRICHTUNG MIT SIGNALFILTERUNG
DISPOSITIF D'ADMINISTRATION DE MÉDICAMENT À FILTRAGE DE SIGNAL

(30) Priority: 01.11.2022 US 202263381775 P
(43) Date of publication of application: 10.09.2025
(73) Proprietor: Eli Lilly and Company, Indianapolis, IN 46285 (US)
(72) Inventor: BOWYER, Andrew Eric, Indianapolis, Indiana 46206-6288 (US); JONES, Richard Earl, JR., Indianapolis, Indiana 46206-6288 (US)
(74) Representative: J A Kemp LLP
(86) International application number: PCT/US2023/078133
(87) International publication number: WO 2024/097624

(56) References cited:
- EP-A2- 3 932 455
- WO-A1-2020/176319
- US-A1- 2019 091 409
- US-A1- 2020 197 614

## Description

### BACKGROUND

Patients suffering from various diseases must frequently inject themselves with medication. To allow a person to conveniently and accurately self-administer medicine, a variety of devices broadly known as pen injectors or injection pens have been developed. Generally, these pens are equipped with a cartridge including a piston and containing a multidose quantity of liquid medication. A drive member is movable forward to advance the piston in the cartridge to dispense the contained medication from an outlet at the distal cartridge end, typically through a needle.

Such devices may have components that physically interact with one another to result in a state change or an action by the device. For example, the device may have a dose button that may be rotated to a set dose and/or actuated to deliver a dose.

Such devices can include electronics, such as an integrated circuit with a processing unit and other components. For example, the electronics can include a sensing device in communication with a processing unit to detect the occurrence of such interactions.

US 2020/197614 A1 discloses a data collection device for attachment to an injection device, such as an injector pen, that includes a sensor arrangement to detect movement of a movable component of the injection device relative to the data collection device during delivery of a medicament by the injection device, and a processor arrangement configured to, based on said detected movement, determine a medicament dosage administered by the injection device. The processor arrangement may monitor the time that has elapsed since the medicament dosage was administered, and control a display to show the medicament dosage and elapsed time to provide a memory aid to the user. In an example embodiment, the sensor arrangement includes an optical encoder and the movable component includes a plurality of light barrier formations. The movable component may be a number sleeve that provides a visual indication of a dose programmed into the injection device.

### SUMMARY

According to an exemplary embodiment of the present disclosure, a method for filtering a signal output from a sensor of a medication delivery device is provided. The sensor is operable to transition between an engaged state in which the signal output from the sensor is in a first logic state and a disengaged state in which the signal output from the sensoeer is in a second logic state. The method includes detecting a first transition of the signal to the first logic state from the second logic state, the first transition occurring at a first time point; determining whether, within a first time period beginning at the first time point, the signal is in the first logic state for a cumulative time period that equals or exceeds a first threshold duration; and determining that the sensor of the medication delivery device transitioned from said disengaged state to said engaged state at the first time point when the cumulative time period equals or exceeds the first threshold duration.

According to another embodiment of the present disclosure, a medication delivery device is provided. The medication delivery device includes a housing comprising a reservoir sized sufficiently to hold medication. The medication delivery device includes a printed circuit board. The medication delivery device includes a sensor mounted to the printed circuit board and operable to output a signal, wherein the sensor is operable to transition between an engaged state in which the signal output from the sensor is in a first logic state and a disengaged state in which the signal output from the sensor is in a second logic state. The medication delivery device includes a microcontroller in electrical communication with the sensor through a logic input to the microcontroller. The microcontroller is configured to: receive the signal output from the sensor; and determine, based on the received signal, whether the sensor has transitioned between said disengaged state and said engaged state at least in part by: determining whether, within a first time period beginning at a first time point corresponding to a first transition of the signal to the first logic state from the second logic state, the signal is in the first logic state for a cumulative time period that equals or exceeds a first threshold duration; and determining that the sensor has transitioned from said disengaged state to said engaged state when the cumulative time period equals or exceeds the first threshold duration.

### BRIEF DESCRIPTION OF DRAWINGS

Additional embodiments of this disclosure, as well as features and advantages thereof, will become more apparent by reference to the description herein taken in conjunction with the accompanying drawings. The components in the figures are not necessarily to scale. Moreover, in the figures, like-referenced numerals designate corresponding parts throughout the different views.
FIG. 1 is a perspective view of a medication delivery device having a dose detection system according to aspects of the present disclosure.
FIG. 2 is a partially exploded perspective view of the medication delivery device of FIG. 1, showing a dose button having a support and a cover, where the cover is shown separated from the support.
FIG. 3 is a partially exploded perspective view of the medication delivery device of FIG. 1 showing the components of the dose detection system.
FIG. 4 is a cross-sectional view of the medication delivery device of FIG. 1.
FIG. 5 is a partial cutaway view of a proximal end of the medication delivery device of FIG. 1, showing components of the dose detection system.
FIG. 6 is an underside view of a portion of the dose button of FIG. 1, showing a printed circuit board held within the dose button cover.
FIG. 7 is an exploded view of the portion of the dose button shown in FIG. 6.
FIG. 8 is a perspective view of a flange of a dose detection system of a medication delivery device.
FIG. 9 is a top down view of the flange of FIG. 8.
FIG. 10 is a perspective view of a dose button support.
FIG. 11 is a top down view of the dose button support of FIG. 10.
FIG. 12 is an exemplary schematic diagram of a printed circuit board, according to some embodiments.
FIG. 13 is an exemplary plot showing the signal received from the sensor of a medication delivery device, according to some embodiments.
FIG. 14 is an exemplary schematic diagram of a printed circuit board having a resistor-capacitor (RC) circuit, according to some embodiments.
FIG. 15 is an exemplary plot showing a signal received from the sensor of a medication delivery device and filtered using an RC circuit, according to some embodiments.
FIGS. 16A-16B is a flowchart showing an exemplary method for filtering a signal received from the sensor of a medication delivery device, according to some embodiments.
FIG. 17 is an exemplary schematic diagram showing a system for detecting transitions of a signal received from the sensor of a medication delivery device, according to some embodiments.
FIG. 18 is an exemplary plot showing an integration of a signal received from the sensor of a medication delivery device, according to some embodiments.

### DETAILED DESCRIPTION

For the purposes of promoting an understanding of the principles of the present disclosure, reference will now be made to the embodiments illustrated in the drawings, and specific language will be used to describe the same. It will nevertheless be understood that no limitation of the scope of the invention is thereby intended.

Provided herein are techniques for filtering a signal received from a sensor of a medication delivery device. According to some embodiments, the sensor is operable to transition between an engaged state and a disengaged state. For example, the sensor may transition to an engaged state when it begins to interact with a sensed component of a medication delivery device. The sensor may transition to a disengaged state when it no longer interacts with the sensed component of the medication delivery device.

The engaged and/or disengaged state(s) can be used to determine information about the medication delivery device, such as dosing information. In some embodiments, the transitions of the sensor between the engaged state and the disengaged state may be used to determine a dosage of medication that has been delivered using the medication delivery device. As a nonlimiting example, the dosage of the medication may be determined based on the number of times that the sensor transitioned to an engaged state and/or to a disengaged state. Accordingly, the inventors have appreciated that it is important to accurately account for the number of times that the sensor has transitioned to the engaged state and/or disengaged state in order to accurately determine the dosage of medication delivered using the medication delivery device. For example, a medication delivery device may include a flange with teeth that rotates as a dose is being dispensed by the device. As the flange rotates, the teeth can interact with a mechanical switch to trigger the switch. Every time the switch is physically triggered by a tooth (e.g., when the switch contacts a tooth and/or when the switch is no longer contacting the tooth), the switch can output an electrical signal that is counted by a processor of the medication delivery device. The processor can count these electrical signals to determine how much the flange has rotated and optionally, based on the rotation information, determine how much insulin was dispensed by the medication delivery device. Alternatively, the processor may communicate the rotation information to another device which determines, based on the rotation information, how much insulin was dispensed by the medication delivery device.

In some embodiments, a signal output by the sensor may be used to infer whether the sensor has transitioned between the engaged state and/or the disengaged state. For example, when the sensor is in the engaged state, it may be configured to output a signal in a first logic state, and when the sensor is in the disengaged state, it may be configured to output a signal in a second logic state that is different from the first logic state. Accordingly, when the sensor transitions between the engaged state and the disengaged state, the signal output by the sensor may transition between the first logic state and the second logic state. For example, the first logic state of the signal may be an asserted state (e.g., logic 1, high state, etc.), while the second logic state of the signal may be a de-asserted state (e.g., logic 0, low state, etc.), or vice versa. While embodiments herein have been described assuming that the sensor outputs a signal in an asserted state when in the engaged state and outputs a signal in a de-asserted state when in the disengaged state, one could easily reverse this with appropriate modifications. For example, the signal may be passed through an inverter before being processed to detect transitions between logic states.

However, the inventors have recognized that there are limitations to this approach. In particular, there may be noise associated with the transition of the sensor between the engaged state and the disengaged state. For example, as the sensor begins to transition to an engaged state, the interactions between the sensor and the sensed component may fluctuate (e.g., the sensor may momentarily lose contact with the sensed component). As another example, when the sensor transitions to a disengaged state, it may bounce between the disengaged state and the engaged state. As a result, the signal output by the sensor may fluctuate between the first logic state and the second logic state several times, even when the sensor is only undergoing a single transition. Accordingly, counting the number of signal transitions to infer the number of sensor transitions may lead to an overestimation and, in turn, an inaccurate estimation of the dosage delivered using the medication delivery device.

Accordingly, the inventors have developed techniques for filtering a signal received from such a sensor of a medication delivery device that address the above-described limitations of conventional techniques. In some embodiments, the techniques detect transitions of the signal between logic states and use the detected transitions to determine whether the sensor has transitioned to an engaged state and/or a disengaged state. For example, in some embodiments, to determine whether the sensor has transitioned to an engaged state at a first time point, the techniques determine a cumulative time period during which the signal was in a first logic state, such as an asserted state, during a first time period beginning at the first time point. If the cumulative time period equals or exceeds a threshold duration, this may indicate that the sensor transitioned to the engaged state at the first time point.

Additionally or alternatively, in some embodiments, the techniques for filtering the signal include processing the signal using one or more hardware components. For example, in some embodiments, the techniques include processing the signal using a resistor-capacitor (RC) circuit. For example, the RC circuit may act as a low-pass filter configured to filter out the high frequency noise caused by the interactions between the sensor and the sensed component as the sensor transitions between the engaged state and/or the disengaged state.

While various embodiments have been described, it will be apparent to those of ordinary skill in the art that many more embodiments and implementations are possible. Accordingly, the embodiments described herein are examples, not the only possible embodiments and implementations. Furthermore, the advantages described above are not necessarily the only advantages, and it is not necessarily expected that all of the described advantages will be achieved with every embodiment.

Devices described herein may comprise a medication, such as for example, within a reservoir or cartridge 20 (described below). In another embodiment, a system may comprise one or more devices including device 10 (described below) and a medication. The term "medication" refers to one or more therapeutic agents including but not limited to insulins, insulin analogs such as insulin lispro or insulin glargine, insulin derivatives, GLP-1 receptor agonists such as dulaglutide or liraglutide , glucagon, glucagon analogs, glucagon derivatives, gastric inhibitory polypeptide (GIP), GIP analogs, GIP derivatives, oxyntomodulin analogs, oxyntomodulin derivatives, therapeutic antibodies and any therapeutic agent that is capable of delivery by the devices described herein. The medication as used in the device may be formulated with one or more excipients. The device is operated in a manner generally as described above by a patient, caregiver or healthcare professional to deliver medication to a person.

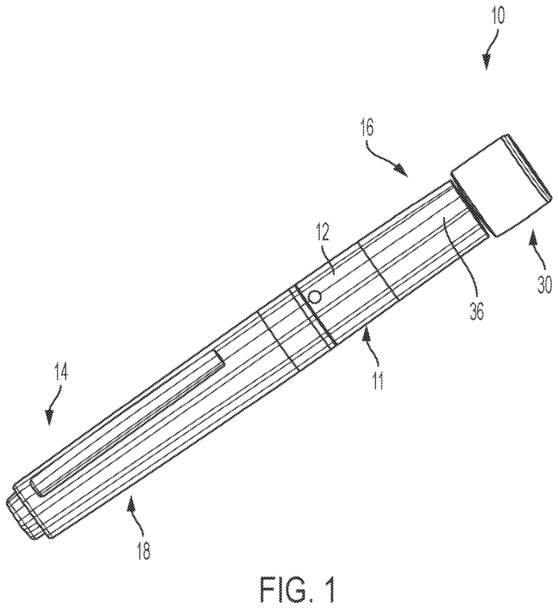

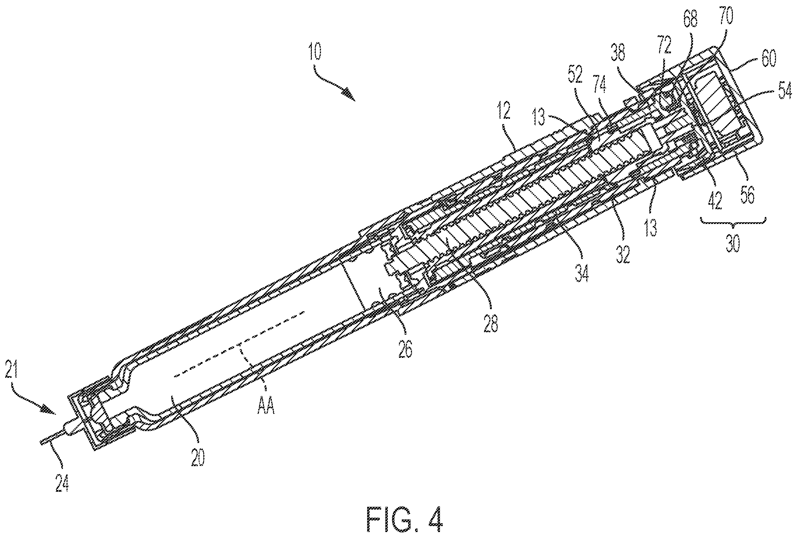

An exemplary medication delivery device 10 is illustrated in FIGS. 1-4 as a pen injector configured to inject a medication into a patient through a needle. Device 10 includes a body 11 that may comprise an elongated, pen-shaped housing 12 including a distal portion 14 and a proximal portion 16. As used herein, the term "distal" refers to the direction and/or portion of a medication delivery device that is pointed towards (or located closer to) the site of injection, while the term "proximal" refers to the direction and/or portion of a medication delivery device that is pointed away from (or located further away from) the site of injection. Distal portion 14 may be received within a pen cap 18. Referring to FIG. 4, distal portion 14 may contain a reservoir or cartridge 20 configured to hold medication to be dispensed through the outlet 21 of the housing a dispensing operation. The outlet 21 of distal portion 14 may be equipped with an injection needle 24. In some embodiments, the injection needle is removable from the housing. In some embodiments, the injection needle is replaced with a new injection needle after each use.

A piston 26 may be positioned in reservoir 20. The medication delivery device may include an injecting mechanism positioned in proximal portion 16 that is operative to advance piston 26 toward the outlet of reservoir 20 during the dose dispensing operation to force the contained medicine through the needled end. The injecting mechanism may include a drive member 28, illustratively in the form of a screw, that is axially moveable relative to housing 12 to advance piston 26 through reservoir 20.

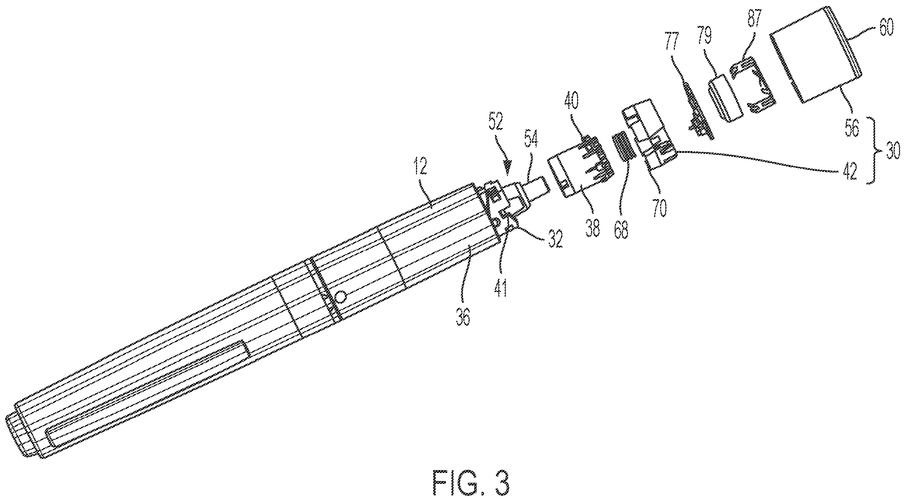

The device may include a dose-setting assembly coupled to the housing 12 for setting a dose amount to be dispensed by device 10. As best seen in FIGS. 3 and 4, in the illustrated embodiment, the dose-setting assembly includes a dose-setting screw 32 and a flange 38. The dose-setting screw 32 is in the form of a screw element operative to spiral (i.e., simultaneously move axially and rotationally) about a longitudinal axis AA of rotation relative to housing 12 during dose setting and dose dispensing. FIGS. 3 and 4 illustrate the dose-setting screw 32 fully screwed into housing 12 at its home or zero dose position. Dose-setting screw 32 is operative to screw out in a proximal direction from housing 12 until it reaches a fully extended position corresponding to a maximum dose deliverable by device 10 in a single injection. The extended position may be any position between a position corresponding to an incremental extended position (such as a dose setting a 0.5 or 1 unit) to a fully extended position corresponding to a maximum dose deliverable by device 10 in a single injection and to screw into housing 12 in a distal direction until it reaches the home or zero position corresponding to a minimum dose deliverable by device 10 in a single injection.

Referring to FIGS. 3 and 4, dose-setting screw 32 includes a helically threaded outer surface that engages a corresponding threaded inner surface 13 of housing 12 to allow dose-setting screw 32 to spiral (i.e. simultaneously rotate and translate) relative to housing 12. Dose-setting screw 32 further includes a helically threaded inner surface that engages a threaded outer surface of sleeve 34 (FIG. 4) of device 10. The outer surface of dose-setting screw 32 includes dose indicator markings, such as numbers that are visible through a dosage window 36 to indicate to the user the set dose amount.

As mentioned above, in some embodiments, the dose-setting assembly further includes a tubular flange 38 that is coupled in the open proximal end of dose-setting screw 32 and is axially and rotationally locked to the dose-setting screw 32 by protrusions 40 received within openings 41 in the dose-setting screw 32. The protrusions 40 of the flange 38 can be seen in FIGS. 3, 8 and 9, and the openings 41 of the dose-setting screw 32 can be seen in FIG. 3.

As seen in FIGS. 3 and 4, delivery device 10 may include an actuator assembly having a clutch 52 and a dose button 30. The clutch 52 is received within the dose-setting screw 32, and the clutch 52 includes an axially extending stem 54 at its proximal end. The dose button 30 of the actuator assembly is positioned proximally of the dose-setting screw 32 and flange 38. Dose button 30 includes a support 42, also referred to herein as an "under button," and a cover 56, also referred to herein as an "over button." As will be discussed, the support 42 and cover 56 enclose electronics components used to store and/or communicate data relating to amount of dose delivered by a medication delivery device.

The support 42 of the dose button may be attached to the stem 54 of the clutch 52, such as with an interference fit or an ultrasonic weld, so as to axially and rotatably fix together dose button 30 and clutch 52.

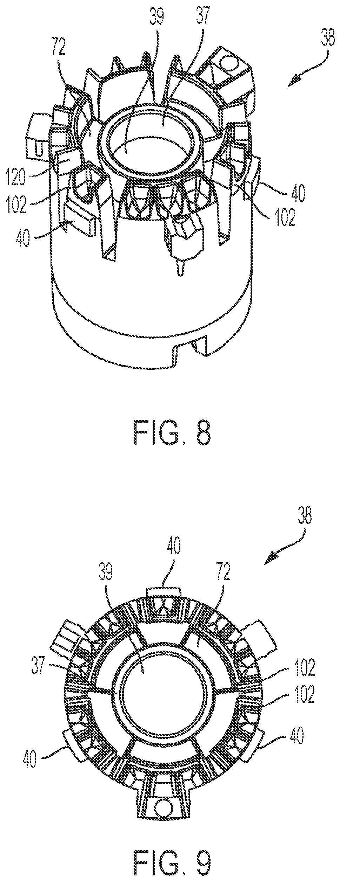

In some embodiments, a portion of the clutch may pass through a lumen 39 of the flange 38. The lumen 39 of the flange is best seen in FIGS. 8 and 9. The lumen 39 may, in some embodiments, serve to help center the clutch 52 in place.

Proximal face 60 of the dose button 30 may serve as a push surface against which a force can be applied manually, i.e., directly by the user to push the actuator assembly (dose button 30 and clutch 52) in a distal direction. A bias member 68, illustratively a spring, may be disposed between the distal surface 70 of support 42 and a proximal surface 72 of tubular flange 38 (FIGS. 8 and 9) to urge the support 42 of the actuation assembly and the flange 38 of the dose-setting assembly axially away from each other. Dose button 30 is depressible by a user to initiate the dose dispensing operation. In some embodiments, the bias member 68 is seated against this proximal surface 72 and may surround a raised collar 37 of the flange 38.

Delivery device 10 is operable in a dose setting mode and a dose dispensing mode. **In** the dose setting mode of operation, the dose button 30 is rotated relative to housing 12 to set a desired dose to be delivered by device 10. **In** some embodiments, rotating the dose button 30 in one direction relative to the housing 12 causes the dose button 30 to axially translate proximally relative to the housing 12, and rotating the dose button 30 in the opposite direction relative to the housing 12 causes the dose button 30 to axially translate distally relative to the housing. **In** some embodiments, clockwise rotation of the dose button moves the dose button 30 distally, and counter-clockwise rotation of the dose button moves the dose button proximally, or vice versa.

**In** some embodiments, rotating the dose button 30 to axially translate the dose button 30 in the proximal direction serves to increase the set dose, and rotating the dose button 30 to axially translate the dose button 30 in the distal direction serves to decrease the set dose. The dose button 30 is adjustable in pre-defined rotational increments corresponding to the minimum incremental increase or decrease of the set dose during the dose setting operation. The dose button may include a detent mechanism such that each rotational increment produces an audible and/or tactile "click." For example, one increment or "click" may equal one-half or one unit of medication.

**In** some embodiments, the set dose amount may be visible to the user via the dial indicator markings shown through a dosage window 36. During the dose setting mode, the actuator assembly, which includes the dose button 30 and clutch 52, moves axially and rotationally with the dose-setting assembly, which includes the flange 38 and the dose-setting screw 32.

Dose-setting screw 32 and flange 38 are fixed rotationally to one another, and rotate and move proximally during dose setting, due to the threaded connection of the dose-setting screw 32 with housing 12. During this dose setting motion, the dose button 30 is rotationally fixed relative to the flange 38 and the dose-setting screw 32 by complementary splines 74 of flange 38 and clutch 52 (FIG. 4), which are urged together by the bias member 68. **In** the course of dose setting, the dose-setting screw 32, flange 38, clutch 52, and dose button 30 move relative to the housing 12 in a spiral manner (i.e., simultaneous rotation and axial translation) from a "start" position to an "end" position. This rotation and translation relative to the housing is in proportion to the amount of dose set by operation of the medication delivery device 10.

Once the desired dose is set, device 10 is manipulated so the injection needle 24 properly penetrates, for example, a user's skin. The dose dispensing mode of operation is initiated in response to an axial distal force applied to the proximal face 60 of dose button 30. The axial force is applied by the user directly to dose button 30. This causes axial movement of the actuator assembly (dose button 30 and clutch 52) in the distal direction relative to housing 12.

The axial shifting motion of the actuator assembly compresses biasing member 68 and reduces or closes the gap between dose button 30 and the tubular flange 38. This relative axial movement separates the complementary splines 74 on clutch 52 and flange 38, and thereby disengages the dose button 30 from being rotationally fixed to the flange 38 and the dose-setting screw 32. In particular, the dose-setting screw 32 is rotationally uncoupled from the dose button 30 to allow backdriving rotation of the dose-setting screw 32 relative to the dose button 30 and the housing 12. Also, while the dose-setting screw 32 and flange 38 are free to rotate relative to the housing 12, the dose button 30 is held from rotating relative to the housing 12 by the user's engagement of dose button 30 by pressing against it.

As dose button 30 and clutch 52 are continued to be axially plunged without rotation relative to housing 12, dose-setting screw 32 screws back into housing 12 as it spins relative to dose button 30. The dose markings that indicate the amount still remaining to be injected are visible through window 36. As dose-setting screw 32 screws down distally, drive member 28 is advanced distally to push piston 26 through reservoir 20 and expel medication through needle 24.

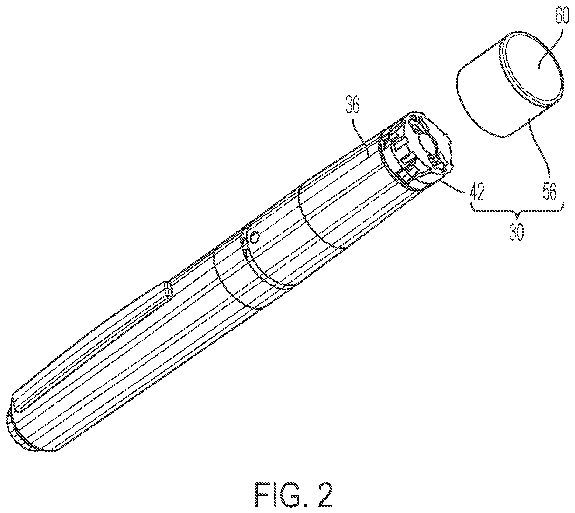

During the dose dispensing operation, the amount of medicine expelled from the medication delivery device is proportional to the amount of rotational movement of the dose-setting screw 32 relative to the housing 12 as the dose-setting screw 32 screws back into housing 12. In some embodiments, because the dose button 30 is rotationally fixed relative to the housing 12 during the dose dispensing mode (e.g., due to engagement with the user's finger), the amount of medicine expelled from the medication delivery device may be viewed as being proportional to the amount of rotational movement of the dose-setting screw 32 relative to the dose button 30 as the dose-setting 32 screws back into housing 12. The injection is completed when the internal threading of dose-setting screw 32 has reached the distal end of the corresponding outer threading of sleeve 34 (FIG. 4). Device 10 is then once again arranged in a ready state or zero dose position as shown in FIGS. 2 and 4.

As discussed above, the dose delivered may be derived based on the amount of rotation of the dose-setting assembly (flange 38 and dose-setting screw 32) relative to the actuator assembly (clutch 52 and dose button 30) during dose delivery. This rotation may be determined by detecting the incremental movements of the dose-setting assembly which are "counted" as the dose-setting assembly is rotated during dose delivery.

Further details of the design and operation of an exemplary delivery device 10 may be found in U.S. Patent No. 7,291,132, entitled Medication Dispensing Apparatus with Triple Screw Threads for Mechanical Advantage. Another example of the delivery device is an auto-injector device that may be found in U.S. Patent No. 8,734,394, entitled "Automatic Injection Device With Delay Mechanism Including Dual Functioning Biasing Member,", where such device being modified with one or more various sensor systems described herein to determine an amount of medication delivered from the medication delivery device based on the sensing of relative rotation within the medication delivery device. Another example of the delivery device is a reusable pen device that may be found in U.S. Patent No. 7,195,616, entitled "Medication Injector Apparatus with Drive Assembly that Facilitates Reset,", where such device being modified with one or more various sensor systems described herein to determine an amount of medication delivered from the medication delivery device based on the sensing of relative rotation within the medication delivery device.

Described herein is a dose detection system that may be operable to determine the amount of dose delivered based on relative rotation between a dose setting member and the device body. The dose detection system utilizes a dose setting member attached to the device body and rotatable relative to the device body about an axis of rotation during dose delivery. A sensed element is attached to and rotationally fixed with the dose setting member. An actuator is attached to the device body and is held against rotation relative to the device body during dose delivery. The sensed element thereby rotates relative to the actuator during dose delivery in relation to the amount of dose delivered.

**In** some embodiments, the dose detection system comprises a rotational sensor attached to the actuator assembly and a sensed element that includes surface features that are equally radially spaced about the axis of rotation of the sensed element.

**In** some embodiments, the dose detection systems may include a sensor and a sensed component attached to components of the medication delivery device. The term "attached" encompasses any manner of securing the position of a component to another component or to a member of the medication delivery device such that they are operable as described herein. For example, a sensor may be attached to a component of the medication delivery device by being directly positioned on, received within, integral with, or otherwise connected to, the component. Connections may include, for example, connections formed by frictional engagement, splines, a snap or press fit, sonic welding or adhesive.

The term "directly attached" is used to describe an attachment in which two components, or a component and a member, are physically secured together with no intermediate member, other than attachment components. An attachment component may comprise a fastener, adapter or other part of a fastening system, such as a compressible membrane interposed between the two components to facilitate the attachment. A "direct attachment" is distinguished from attachment where the components/members are coupled by one or more intermediate functional members.

The term "fixed" is used to denote that an indicated movement either can or cannot occur. For example, a first member is "fixed rotationally" with a second member if the two members are required to move together in rotation. **In** one aspect, a member may be "fixed" relative to another member functionally, rather than structurally. For example, a member may be pressed against another member such that the frictional engagement between the two members fixes them together rotationally, while the two members may not be fixed together absent the pressing of the first member.

Various sensor arrangements are contemplated herein. **In** general, the sensor arrangements comprise a sensor and a sensed component. The term "sensor" refers to any component which is able to detect the relative position or movement of the sensed component. The sensor may be used with associated electrical components to operate the sensor. The "sensed component" is any component for which the sensor is able to detect the position and/or movement of the sensed component relative to the sensor. For the dose detection system, the sensed component rotates relative to the sensor, which is able to detect the rotational movement of the sensed component. The sensor may comprise one or more sensing elements, and the sensed component may comprise one or more sensed elements. The sensor detects the movement of the sensed component and provides outputs representative of the movement of the sensed component.

Illustratively, the dose detection system includes an electronics assembly suitable for operation of the sensor arrangement as described herein. The medication delivery device may include a controller that is operably connected to the sensor to receive outputs from the sensor. The controller begins receiving generated signals from the sensor indicative of counts from first to last one for a total number of counts that is used for determining total displacement, e.g. angular displacement. In the case of detecting an angular movement of a dose-setting assembly, the controller may be configured to receive data indicative of the angular movement of the dose-setting assembly that can be used to determine from the outputs the amount of dose delivered by operation of the medication delivery device. The controller may, optionally, be configured to determine from the outputs the amount of dose delivered by operation of the medication delivery device. The controller may include conventional components such as a processor, power supply, memory, microcontrollers, etc. Additionally or alternatively, at least some components may be provided separately, such as by means of a computer, smart phone or other device. Means are then provided to operably connect the external controller components with the sensor at appropriate times, such as by a wired or wireless connection. For example, the controller on board the medication delivery device may be configured to determine only the amount of angular movement of the dose-setting assembly and communicate this angular movement to the external controller. The external controller may then be configured to determine the amount of dose delivered based on the angular movement information.

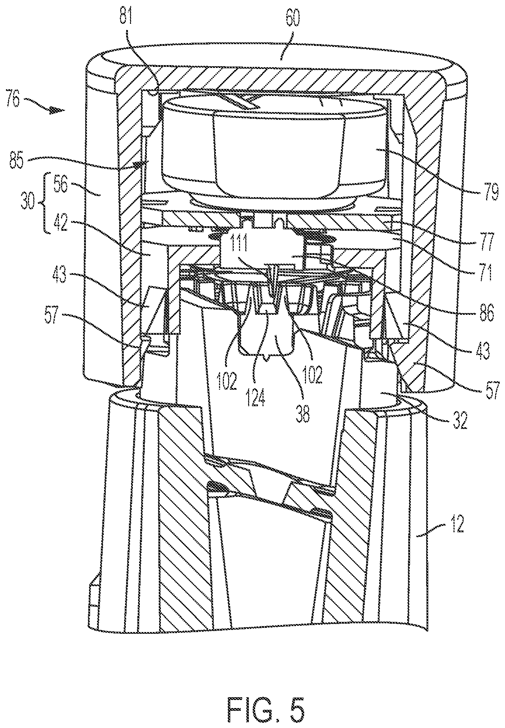

According to one aspect, the electronics assembly includes a sensor arrangement including one or more sensors operatively communicating with a processor for receiving signals from the sensor representative of the sensed rotation. An exemplary electronics assembly 76 is shown in FIGS. 5-7 and can include a sensor 86, and a printed circuit board (PCB) 77 having a plurality of electronic components. The printed circuit board may be a flexible printed circuit board. The circuit board of the electronics assembly 76 may include a microcontroller unit (MCU) as the controller comprising at least one processing core and internal memory. The electronics assembly may include a power source 79, e.g. a battery, illustratively a coin cell battery, for powering the components. The controller of electronics assembly 76 may include control logic operative to perform the operations described herein, including detecting the angular movement of the dose-setting assembly during dose setting and/or dose delivery and/or detecting a dose delivered by medication delivery device 10 based on a detected rotation of the dose-setting assembly relative to the actuator assembly. Many, if not all of the components of the electronics assembly, may be contained in a compartment 85 within the dose button 30. In some embodiments, the compartment 85 may be defined between a proximal surface 71 of support 42 of the dose button and a distal surface 81 of the cover 56 of the dose button. In the embodiment shown in FIG. 5, the electronics assembly 76 is permanently integrated within the dose button 30 of the delivery device. In other embodiments, the electronics assembly is provided as a module that can be removably attached to the actuator assembly of the medication delivery device.

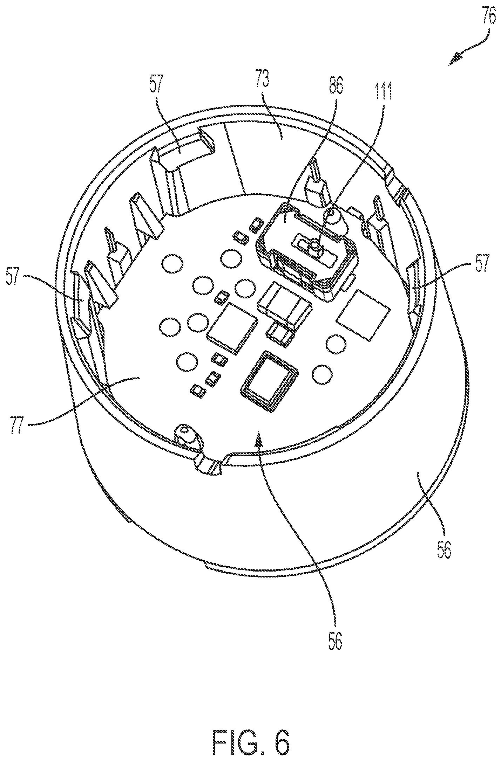

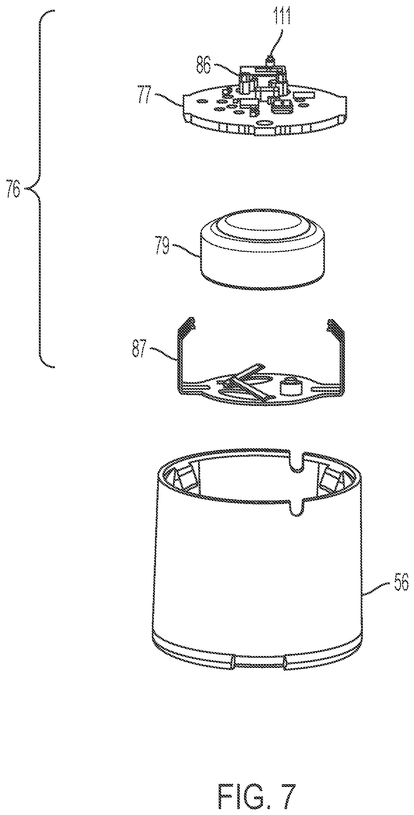

An underside view of the electronics assembly 76 held within the cover 56 is shown in FIG. 6, and an exploded view of the electronics assembly 76 is shown in FIG. 7. As shown in FIGS. 6 and 7, the electronics assembly 76 may include a printed circuit board (PCB) 77 and a sensor 86 having a contact surface 111. As shown in FIG. 7, the electronics assembly 76 may also include a battery 79 and a battery cage 87.

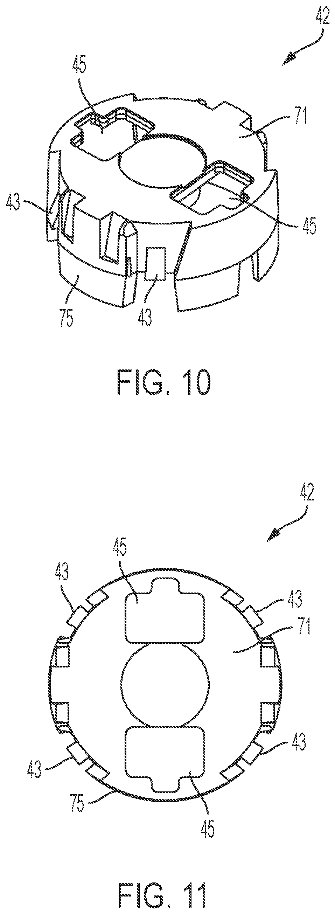

In some embodiments, at least a portion of the sensor 86 extends out of the compartment 85 of the dose button 30. As best seen in FIGS. 10 and 11, the support 42 of the dose button 30 may include one or more openings 45 through which the sensor 86 can extend through. In some embodiments, during assembly of the medication delivery device, the contact surface 111 of the sensor 86 is passed through the opening 45 of the support 42. This may permit the contact surface 111 of the sensor to interact with a component that is external to the compartment 85 of the dose button 30. In some embodiments, while only one of the openings 45 in the support 42 is needed to accommodate a sensor, a second opening may be provided, e.g. for symmetry of the support component, which help with manufacturing of the component and/or assembly of the component with the medication delivery device.

The controller of electronics assembly 76 may be operative to store the total angular movement used for determining dose delivery and/or the detected dose delivery in local memory (e.g., internal flash memory or on-board EEPROM). The controller may be further operative to wirelessly transmit a signal representative of the total counts, total angular movement, and/or detected dose to an external device, such as a user's mobile device or a remote server. Transmission may, for example, be over a Bluetooth low energy (BLE) or other suitable short or long range wireless communication protocol. Illustratively, the BLE control logic and controller are integrated on the same circuit.

As discussed, according to one aspect, the dose detection system involves detecting relative rotational movement between two assemblies of the medication delivery device. With the extent of rotation having a known relationship to the amount of a delivered dose, the sensor operates to detect the amount of angular movement from the start of a dose injection to the end of the dose injection. For example, in some embodiments, the relationship for a pen injector is that an angular displacement of a dose-setting assembly of 18° is the equivalent of one unit of dose, although other angular relationships are also suitable, such as, for example, 9, 10, 15, 20, 24 or 36 degrees may be used for a unit or a half unit. The sensor system is operable to determine the total angular displacement of a dose setting member during dose delivery. Thus, if the angular displacement is 90°, then 5 units of dose have been delivered.

The angular displacement is determined by counting increments of dose amounts as the injection proceeds. For example, a sensing system may use a repeating pattern of a sensed element, such that each repetition is an indication of a predetermined degree of angular rotation. Conveniently, the pattern may be established such that each repetition corresponds to the minimum increment of dose that can be set with the medication delivery device.

The dose detection system components may be permanently or removably attached to the medication delivery device. In some embodiments, at least some of the dose detection system components are provided in the form of a module that is removably attached to the medication delivery device. In other embodiments, the dose detection system components are permanently attached to the medication delivery device.

In some embodiments, a sensor may detect, during dose delivery, the relative rotation of a sensed component that is rotationally fixed to the dose-setting screw 32, from which is determined the amount of a dose delivered by the medication delivery device. In an illustrative embodiment, a rotational sensor is attached, and rotationally fixed, to the actuator assembly. The actuator assembly does not rotate relative to the device housing during dose delivery.

In some embodiments, a sensed component is attached, and rotationally fixed, to the dose-setting screw 32, which rotates relative to the dose button 30 and the device housing 12 during dose delivery. In some of the embodiments described herein, the sensed component includes a ring structure having a plurality of proximally extending projections circumferentially disposed relative to one another. Projections are shaped and sized to deflect a movable element of the rotational sensor. One illustrative embodiment of such a sensed component is tubular flange 38, best seen in FIGS. 3, 5, 8, and 9. Embodiments described herein may be provided for a module that is removably attachable to the dose button of the delivery device or integrated within the dose button of the delivery device.

During dose delivery, dose-setting screw 32 is free to rotate relative to dose button 30. In the illustrative embodiment, the electronics assembly 76 is rotationally fixed with the dose button 30 and does not rotate during dose delivery.

As seen in FIGS. 2, 3 and 5, the dose button 30 comprises a cover 56 coupled to a support 42. An electronics assembly 76 may be at least partially contained within a compartment 85 defined between the cover 56 and the support. In some embodiments, the cover and support have corresponding splines that engage with one another to couple the cover and support together. For example, in some embodiments, the cover 56 may couple to the support 42 via one or more snaps 57 on the cover 56 and corresponding to one or more protrusions 43 on the support. As seen in FIG. 5 and 6, the snaps 57 on the cover 56 may be directed radially inwardly from an inner circumferential sidewall 73. As seen in FIGS. 5, 10 and 11, the protrusions 43 on the support 42 may be directed radially outwardly from an outer circumferential sidewall 75 of the support 42. The protrusions 43 may form a triangular ramp shape.

The snaps 57 on the cover 56 are configured to snap over and mate with the protrusions 43 on the support to couple the cover to the support. In some embodiments, the protrusion on the support comprises a continuous annular protrusion around the outer circumferential sidewall of the support. The cover 56 may attach to the support 42 via frictional engagement, interference fit or any other suitable fit. In some embodiments, the cover 56 is permanently fixed to the support 42 during assembly, e.g., via ultrasonic welding, adhesive, or other suitable fixation approach.

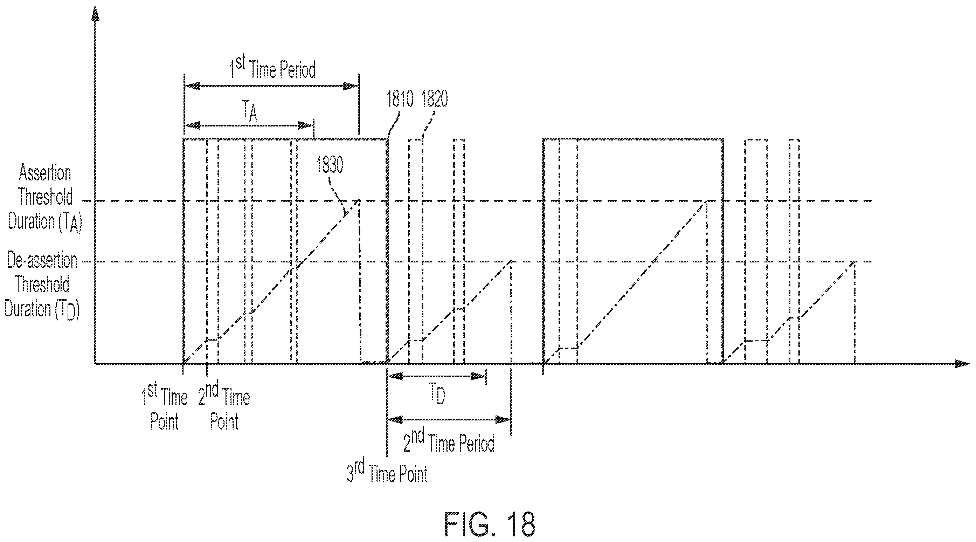

As seen in FIGS. 8 and 9, the tubular flange 38 may include a plurality of axially directed teeth 102 that are equally radially spaced about a rotation axis and arranged to correlate to the equivalent of one unit of dose. In this illustrative embodiment, the tubular flange 38 includes 20 teeth 102 that are equally rotationally spaced from one another, such that the rotation distance between two adjacent teeth corresponds to 18 degrees of rotation. Thus, with the tubular flange 38 of FIG. 8, 18 degrees of rotation of the tubular flange 38 may be used to represent one dosage unit or a half dosage unit. It should be appreciated that, in other embodiments, different total numbers of teeth may be used to create other angular relationships, such as, for example, 9, 10, 15, 18, 20, 24 or 36 degrees may be used for a unit or 0.5 unit.

A recess 124 may be defined between each pair of adjacent teeth 102. Each tooth 102 may have an approximately triangular shaped profile, each having a surface 120 against which a contact surface 111 of a sensor may slide.

In some embodiments, the sensor for detecting rotation of the tubular flange includes a movable element that has a contact portion capable of resting against the teeth of the tubular flange and is spring-biased such that the contact surface is configured to slide against and over the teeth during rotation of the flange relative to the actuator assembly during dose delivery. The sensor is responsive to the movement of the contact portion over the teeth and generates signals corresponding to the flange. A controller is responsive to the signals generated by the sensor to determine a dose count for determining the dosage delivered based on the detected rotation of the flange relative to the actuator assembly during dose delivery.

The contact surface may be biased against the physical features of the tubular flange to ensure proper contact between the contact surface and the physical features during rotation. In one embodiment, the movable element is a resilient member having one portion attached to the actuator at a location displaced from the contact surface. In one example, the movable element is a following member comprising a beam attached at one end to the actuator and having the contact surface at the other end. The beam is flexed to urge the contact surface in the direction of the surface features. Alternatively, the movable element may be biased in any of a variety of other ways. In addition to the use of a resilient beam, the biasing may be provided, for example, by use of a spring component. Such spring component may for example comprise a compression, tension, or torsion coil spring. In yet other embodiments, the movable element may be biased against the surface features of the sensed element by a separate resilient member or spring component bearing against the movable element.

FIG. 5 depicts an illustrative embodiment of a sensor 86 having a contact surface 111 interacting with teeth 102 of a tubular flange 38. As the flange 38 rotates relative to the dose button 30 during delivery, the teeth 102 of the flange contact and slide against the contact surface 111 of the sensor 86, causing the contact surface 111 to move in an oscillating manner. The movement of the contact surface 111 may be a combination of axial and lateral movement as the contact surface 111 slides into and out of the recesses 124 defined between the teeth 102 of the flange 38. The sensor 86 may be configured to track the movement of the contact surface 111 and associate the movement with an output signal that is sent to a controller.

As an alternative to teeth on the tubular flange, surface features that interact with the sensor may comprise anything detectable by the sensor. The sensor arrangement may be based on a variety of sensed characteristics, including tactile, optical, electrical and magnetic properties, for example. In the illustrative embodiments shown in the figures, the surface features are physical features which allow for detection of incremental movements as the dose-setting assembly rotates relative to the actuator assembly. In alternative embodiments, the sensor may be a piezoelectric sensor, a magnetic sensor such as a Hall effect sensor, an accelerometer for detecting vibration, e.g. of a ratcheting or other detent mechanism, where vibration can be correlated with rotational movement, an optical sensor such as a reflective sensor, an interrupter sensor, or an optical encoder, or any other sensor suitable for sensing rotation of a first component relative to a second component.

In some embodiments, when a user presses axially on face 60 of the dose button 30, the dose button 30 advances distally relative to the housing 12, compressing spring 68. Continued pressing of the dose button 30 distally results in back driving of the dose-setting screw 32 in a spiral direction relative to housing 12. As a result, the dose-setting screw 32 and flange 38 are driven to rotate by the axially pressing upon the dose button 30. In some embodiments, the dose detection system is operable for dose detection only while the dose button is being pressed.

In some embodiments, the electronics assembly may include a clock or timer to determine the time elapsed between counts caused by trigger of the rotational sensor from the surface features of the sensed element. When no counts have been detected by the controller after a period of time this may be used to indicate that the dose has completed.

In some embodiments, a single sensing system may be employed for both dose detection sensing and wake-up activation. For example, upon the initial sensing of rotation of the sensed element by the sensor, the controller is configured to allow wake-up or activation of the electronics assembly to a greater or full power state. The wake-up feature is configured to allow power transmission from the power source (shown as battery) for powering up the electronic components for dose sensing in order to minimize inadvertent power loss or usage when a dose dispensing event is not occurring. In other embodiments, a separate wake-up switch may be provided and arranged within the dose button housing and triggered when the dose button is in its distal position. After activation of the electronics assembly, the controller begins receiving generated signals from the rotational sensor indicative of counts from first to last one for a total number of counts that is used for determining total angular displacement and thus the amount of dose delivered.

In some embodiments, the electronics assembly may have a controller that is configured to receive an output signal from a rotational sensor. The controller of the electronics assembly may be programmed to convert the intermediate signal to a conditioned digital signal, which may be a single step/square wave with a predetermined width representing a predetermined time. In some embodiments, output signals that are less than a predetermined level may be filtered out and ignored.

As described herein, the printed circuit board (e.g., the printed circuit board 77) can include various processing circuitry and/or logic that generates data based on the operation of the medication delivery device. For example, the processing circuitry can count the number of times the sensor (e.g., the sensor 86) is activated or triggered during an injection to determine a dose size of the injection (e.g., the dose a particular insulin injection). As described herein, the relative rotational movement between a dose-setting assembly and an actuator of the medication delivery device can be sensed in order to determine the amount of a dose delivered by a medication delivery device, because the sensed relative rotational movements can be correlated to the amount of the dose delivered.

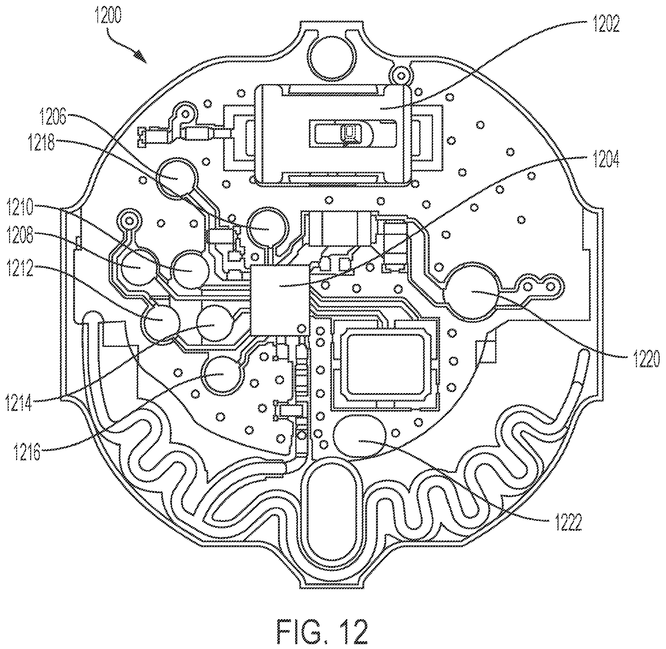

FIG. 12 is an exemplary schematic diagram of a printed circuit board 1200, according to some embodiments. The printed circuit board 1200 (e.g., printed circuit board 77) includes various components, including a sensor 1202 (e.g., sensor 86 in FIG. 6) that is in electrical communication with a microcontroller 1204. The printed circuit board 1200 includes a set of pads 1206, 1208, 1210, 1212, 1214, 1216, 1218, 1220 and 1222 that are in electrical communication with the microcontroller 1204. The pads can be used to connect electrical components to the microcontroller 1204, e.g., for testing, and/or the like. Some of the pads, such as pads 1208, 1210, 1212, 1214, 1216, and 1218, may not be in communication with the microcontroller 1204 by default. For example, the microcontroller may be initially programmed (e.g., via associated registers) such that some of the pads are not in electrical communication with the microcontroller 1204 (e.g., via programmable switches or resistors). One or more of the pads can be placed in electrical communication with a logic input, such as general-purpose input/output (GPIO) pin(s) of the microcontroller 1204. As an example, the microcontroller can be programmed to modify internal programmable components (e.g., one or more pull-up resistors and/or pull-down resistors) to place the pads in electrical communication with the logic input.

In some embodiments, a GPIO pin input to the microcontroller 1204 can be a logic level input. The microcontroller 1204 can detect a logical 1 if a voltage above a certain maximum threshold is applied to a GPIO pin, while the microcontroller 1204 can detect a logical 0 if a voltage below a certain minimum threshold is applied to the GPIO pin. Some pads on the printed circuit board may be connected to a voltage source. For example, pad 1220 can provide a battery voltage Vbat. As another example, pad 1206 can provide a voltage from a DC/DC converter Vdcdc.

As described herein, the microcontroller 1204 (e.g., including based on input from the sensor 1202) may be operative to process dose data and/or other data of the medication delivery device. For example, the microcontroller 1204 can be configured to store the total angular movement used for determining dose delivery and/or the detected dose delivery in local memory (e.g., internal flash memory or on-board EEPROM). The microcontroller 1204 may be further operative to wirelessly transmit a signal representative of the total counts, total angular movement, and/or detected dose to an external device, such as a user's mobile device or a remote server (e.g., via BLE control logic and controller integrated on the printed circuit board 1200).

As described herein, in some embodiments, a medication delivery device includes a sensor (e.g., sensor 86 in FIG. 6, sensor 1202 in FIG. 12) that detects the movement of the sensed component and provides output representative of the movement of the sensed component. For example, the sensor may generate a signal indicative of whether the sensor is in an engaged state or a disengaged state.

In some embodiments, the state of the sensor depends on physical contact between the sensor and the sensed component. For example, the sensor may be considered to be in an engaged state when it is in physical contact with the sensed component or with an intermediate component positioned between the sensor and the sensed component. By contrast, the sensor may be considered to be in a disengaged state when the sensor is not in physical contact with the sensed component or with the intermediate component positioned between the sensor and the sensed component. As a nonlimiting example, the sensor may be in an engaged state when a contact portion of the sensor contacts a tooth (e.g., teeth 102 in FIGS. 8-9) of a tubular flange (e.g., flange 38 in FIGS. 5 and 8-9) of the medication delivery device. The sensor may be in a disengaged state when the contact portion of the sensor is not in contact with the tooth, such as when the contact portion is positioned in the recesses (e.g., recesses 124 in FIG. 5) between the teeth of the tubular flange.

Additionally or alternatively, in some embodiments, the state of the sensor depends on the sensor arrangement. As described herein, the sensor arrangement may be based on a variety of sensed characteristics, including tactile, optical, electrical, and magnetic properties, for example. The sensor may be a piezoelectric sensor, a magnetic sensor such as a Hall effect sensor, an accelerometer for detecting vibration, an optical sensor, an interrupter sensor, or an optical encoder, or any other sensor suitable for sensing rotation of a first component relative to a second component. Therefore, it should be appreciated that the sensor may be considered to be in an engaged state when it senses any suitable sensed characteristic and that the sensor may be considered to be in a disengaged state when it does not sense the sensed characteristic, or vice versa.

In some embodiments, the sensor generates a signal in response to its interactions with a sensed component. For example, when the contact portion of a sensor is in contact with a tooth of a tubular flange (e.g., the sensor is in an engaged state), the sensor may generate a signal that is in a first logic state. When the contact portion of the sensor is not in contact with the sensed component (e.g., when the contact portion is positioned in the recesses between teeth, or when the sensor is in a disengaged state), the sensor may generate a signal that is in a second logic state, different from the first logic state. For example, the first logic state of the signal may be an asserted state (e.g., logic 1, high state, etc.), while the second logic state of the signal may be a de-asserted state (e.g., logic 0, low state, etc.), or vice versa.

Accordingly, in some embodiments, the generated signal can be used to determine the rotation of the sensor with respect to the sensed component. Continuing with the example of the tubular flange (e.g., tubular flange 38), when the signal transitions between a first logic state and a second logic state five times, this may indicate that a contact portion of the sensor slid against and over five teeth of the medication delivery device. Given the separation between said teeth, it may be possible to determine the rotation of the dose setting assembly within the medication delivery device. As described herein, the rotation of the dose setting assembly may then be used to determine a dosage of medication delivered using the medication delivery device. The dosage of medication may be determined by the controller of the medication delivery device, or by a separate device in communication with the medication delivery device, based on data indicative of the rotation of the dose setting assembly.

However, the signal output by the sensor can be noisy, such that it can be challenging to use the signal to determine whether the sensor is in an engaged state or a disengaged state. For example, as a sensor begins to engage with a sensed component, it may momentarily lose contact with the sensed component, such that the signal output by the sensor momentarily transitions to the second logic state when it should have remained in the first logic state. For example, the leading edge of the contact portion of a sensor may bounce when it begins to slide over the tooth of a tubular flange, causing it to momentarily lose contact with the tooth.

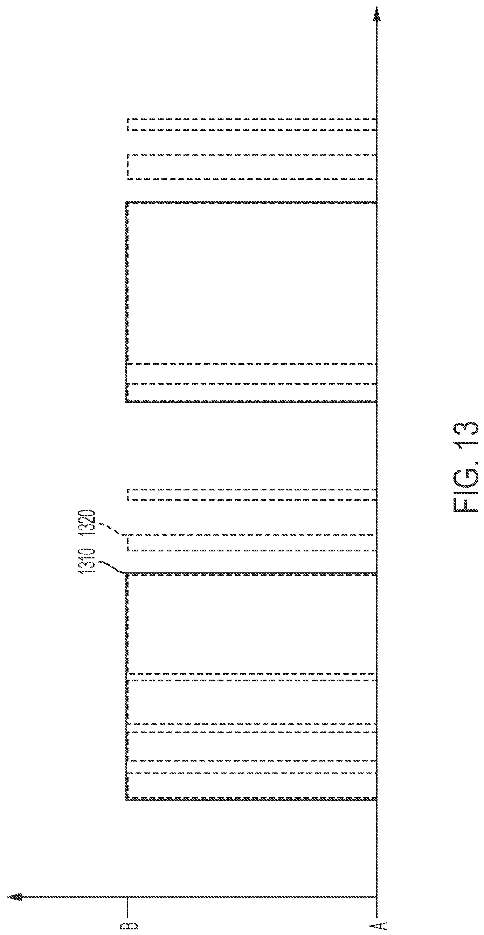

FIG. 13 is an exemplary plot showing the signal received from the sensor of a medication delivery device, according to some embodiments. Line 1310 indicates the times at which a sensor of a medication delivery device actually transitioned between an engaged state (point "B" along the y-axis) and a disengaged state (point "A" along the y-axis). For example, this may correspond to the contact portion of the sensor sliding over two teeth of the tubular flange of the medication delivery device. Line 1320 indicates the times at which the signal output by the sensor transitioned between an asserted state (e.g., a first logic state, or point "B" along the y-axis) and a de-asserted state (e.g., a second logic state, or point "A" along the y-axis). As shown, the signal transitioned between an asserted state and a de-asserted state several more times than the switch actually transitioned between an engaged state and a disengaged state. Therefore, counting the number of times that the signal is in an asserted state to infer the number of times the sensor is in an engaged state would lead to an overestimation of this value. This would in turn lead to an inaccurate determination of the rotation of a dose setting assembly, which would result in an inaccurate estimation of the dosage of medication delivered using the medication delivery device.

Accordingly, the inventors have developed techniques for filtering the signal from the sensor, such that the signal can be used to more reliably determine when the sensor has transitioned between an engaged state and a disengaged state. In turn, the techniques can more reliably and accurately estimate the rotation of the dose setting assembly, and more reliably and accurately determine the dosage of medication delivered using the medication delivery device.

In some embodiments, the techniques for filtering the signal include using one or more hardware components. For example, a printed circuit board (e.g., printed circuit board 1200 in FIG. 12) of a medication delivery device may include one or more components used for filtering the signal received from a sensor. As a nonlimiting example, the printed circuit board may include a resistor-capacitor circuit.

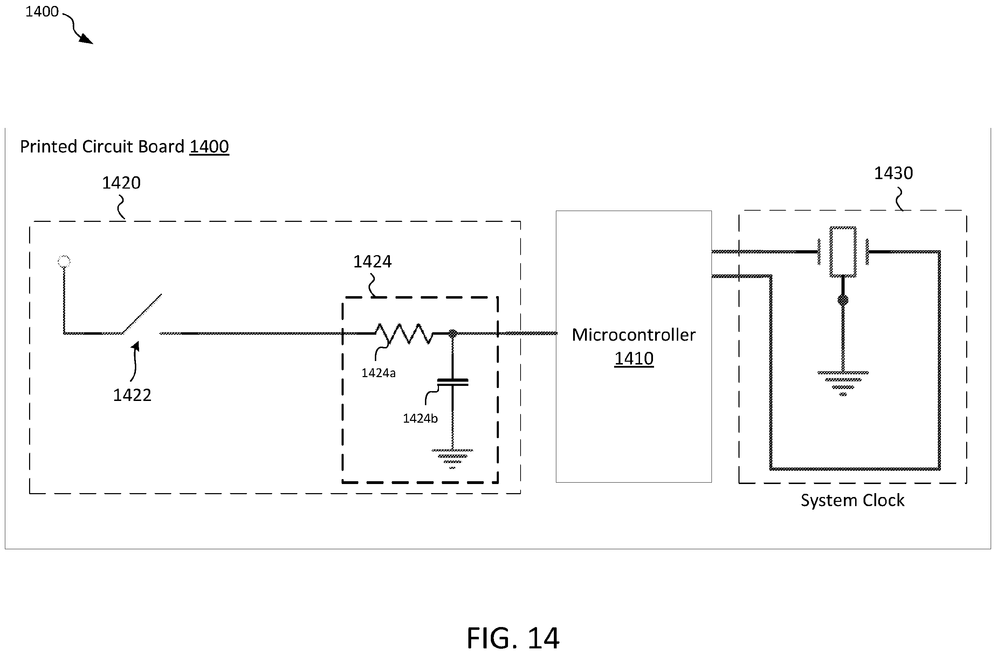

FIG. 14 is an exemplary schematic diagram of a printed circuit board having a resistor-capacitor (RC) circuit, according to some embodiments. As shown, the printed circuit board 1400 includes dose detector components 1420 and system clock 1430, each of which are in electrical communication with microcontroller 1410. However, it should be appreciated that the printed circuit board 1400 may include one or more additional or alternative components, such as those described herein including at least with respect to FIGS. 5-7 and 12.

In some embodiments, the system clock 1430 is used by microcontroller 1410 to keep track of time. The system clock 1430 may include an oscillator circuit, the frequency of which may be used to keep track of time. The oscillator circuit may include a resistor-capacitor (RC) oscillator circuit, an inductor-capacitor (LC) oscillator circuit, a crystal oscillator circuit, or any other suitable oscillator circuit, as aspects of the technology described herein are not limited in this respect. However, it should be appreciated that the system clock 1430 is not limited to an oscillator circuit, and may include any other suitable clock, as aspects of the technology are not limited in this respect.

In some embodiments, the dose detector components 1420 include switch 1422 and RC circuit 1424. In some embodiments, switch 1422 comprises a portion of a sensor (e.g., sensor 86 in FIGS. 5-7), which is configured to detect the position and/or movement of a sensed component, such as a tooth of a tubular flange. For example, the switch may be configured to close when a contact portion of the sensor is in physical contact with a sensed component and configured to open when the contact portion is not in physical contact with the sensed component, or vice versa.

Accordingly, in some embodiments, the RC circuit 1424 is configured to receive a signal from the sensor when the switch 1422 is closed and to transmit a filtered signal to microcontroller 1410. In alternative embodiments, the microcontroller 1410 is configured to receive a signal directly from the sensor when the switch 1422 is closed.

In some embodiments, the RC circuit 1424 is configured to filter the signal received from the switch 1422. The RC circuit 1424 may include resistor 1424a and capacitor 1424b. The resistance of resistor 1424a and the capacitance of capacitor 1424b may be selected such that the RC circuit 1424 acts as a low-pass filter. For example, the RC circuit may be configured to filter out noise in the signal, such as the momentary signal de-assertions that occur when the sensor is actually in an engaged state and/or the momentary signal assertions that occur when the sensor is actually in a disengaged state. Accordingly, it should be appreciated that the resistor may be of any suitable resistance and that the capacitor may be of any suitable capacitance, as aspects of the technology are not limited in this respect.

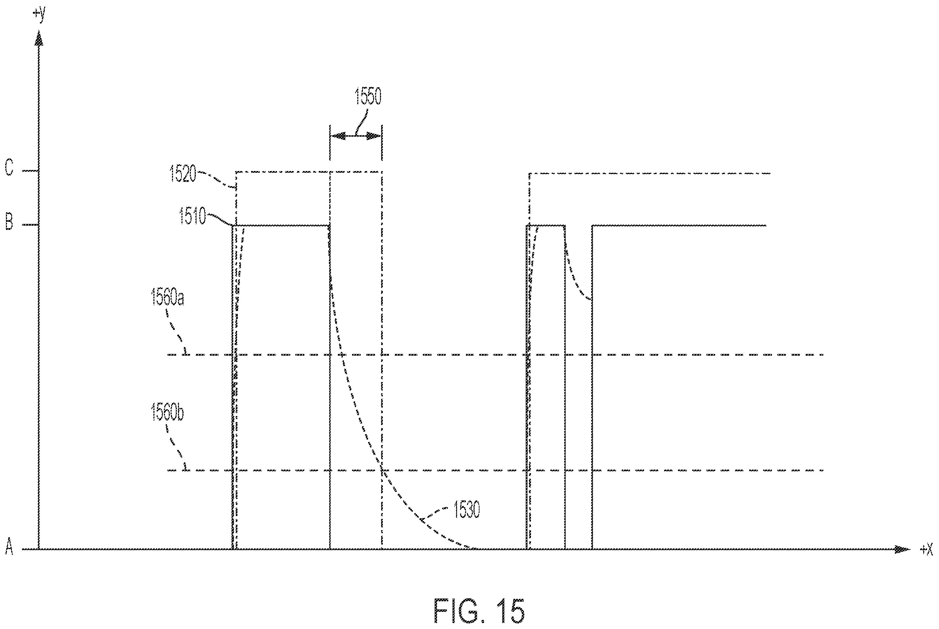

In some embodiments, the RC circuit is configured to act as a low-pass filter with respect to only one or both types of signal transitions (e.g., transitions of the signal from the second logic state to the first logic state and/or transitions of the signal from the first logic state to the second logic state). For example, as shown in FIG. 15, an RC circuit operates as a low pass filter on transitions of the signal from an asserted state to a de-asserted state. Accordingly, as described herein, the RC circuit in the example of FIG. 15 may be configured to filter out high frequency noise as the sensor transitions to a disengaged state.

In some embodiments, the RC circuit 1424 transmits the filtered signal to the microcontroller 1410. The microcontroller 1410 may include any suitable microcontroller, such as the microcontroller 1204 described herein including at least with respect to FIG. 12. In some embodiments, microcontroller 1410 receives the filtered signal through a GPIO pin input. The GPIO pin input may be a logic level input. The microcontroller 1410 can detect a logical one (1) if a voltage above a certain maximum threshold is applied to a GPIO pin, while the microcontroller 1410 can detect a logical zero (0) if a voltage below a certain minimum threshold is applied to the GPIO pin.

While the RC circuit 1424 may function to filter out unwanted noise, there may be some limitations associated with the use of an RC circuit 1424, as described herein including at least with respect to FIG. 15.

FIG. 15 is an exemplary plot showing a signal received from the sensor of a medication delivery device and filtered using an RC circuit, according to some embodiments. As shown, line 1510 indicates the times at which a sensor of a medication delivery device actually transitioned between an engaged state and a disengaged state. Line 1530 indicates the analog signal output from an RC circuit, such as RC circuit 1424 shown in FIG. 14. Line 1520 indicates the digital signal received and processed by a microcontroller of a medication delivery device, such as microcontroller 1410 shown in FIG. 14.

In some embodiments, when a contact portion of the sensor comes into contact with a sensed component, switch 1422 closes, allowing the RC circuit 1424 to charge. The analog signal 1530 received by the microcontroller, as the RC circuit charges, is indicated by the portions of the analog signal 1530 that transition in the positive y-direction to point "B".

In some embodiments, when the analog signal 1530 exceeds threshold 1560a, the digital signal 1520 transitions to an asserted state. For example, as shown in FIG. 15, when analog signal 1530 crosses threshold 1560a, digital signal 1520 transitions from point "A" to point "C" along the y-axis, which correspond respectively to a de-asserted state and an asserted state of the signal 1520. In some embodiments, threshold 1560a may include any suitable threshold, as aspects of the technology described herein are not limited in this respect.

In some embodiments, when the contact portion of the sensor is no longer in contact with a sensed component, switch 1422 opens, causing the RC circuit 1424 to discharge. The analog signal 1530 received by the microcontroller, as the RC circuit discharges, is indicated by the portions of the analog signal 1530 that transition downwards, from point "B", in the negative y-direction.

In some embodiments, when the analog signal 1530 falls below threshold 1560b, the digital signal 1520 transitions to a de-asserted state. For example, as shown in FIG. 15, when analog signal 1530 falls below threshold 1560b, digital signal 1520 transitions from point "C" to point "A" along the y-axis.

In some embodiments, the RC circuit charges at a faster rate than it discharges. Therefore, as shown in FIG. 15, the microcontroller may continue to receive a non-zero analog signal 1530 after the sensor has transitioned to a disengaged state, as indicated by line 1510. This may also result in a delay, such as delay 1550, between the time when the sensor transitions to a disengaged state and the time when the analog signal 1530 falls below threshold 1560b. The delay may depend on the properties of the RC circuit used to filter the signal received from the switch. For example, delay 1550 may range from 20-120 µs.

In some embodiments, signal transition delays do not affect the results of downstream processing of signal 1520. For example, the first assertion of digital signal 1520 corresponds to one occurrence of the sensor transitioning to the engaged state. Even though there is a delay 1550, the delay 1550 does not affect the calculation of the number of occurrences of the sensor transitioning to the engaged state during the time period when digital signal 1520 was in an asserted state.

However, in some embodiments, signal transition delays do affect the results of downstream processing of signal 1520. For example, the second assertion of digital signal 1520 corresponds to two occurrences of the sensor transitioning to the engaged state. As shown, because the RC circuit discharges too slowly after the sensor transitions from the engaged state to the disengaged state, analog signal 1530 decreases, but does not decrease below threshold 1560b before the sensor transitions back to the engaged state. Accordingly, the digital signal 1520 does not transition to a de-asserted state and, as a result, cannot be used to reliably detect both occurrences of the sensor transitioning to the engaged state.

In some embodiments, this limitation may be addressed by adjusting the RC circuit to allow higher frequencies to pass (e.g., such that line 1530 follows line 1510 more closely). However, such an adjustment may also allow more noise to pass through the RC circuit, resulting in an inaccurate estimate of both the number of times the sensor transitioned to an engaged state and the dosage of medication delivered by the medication delivery device.

Accordingly, the inventors have developed additional or alternative techniques for filtering a signal received from the sensor of a medication delivery device.

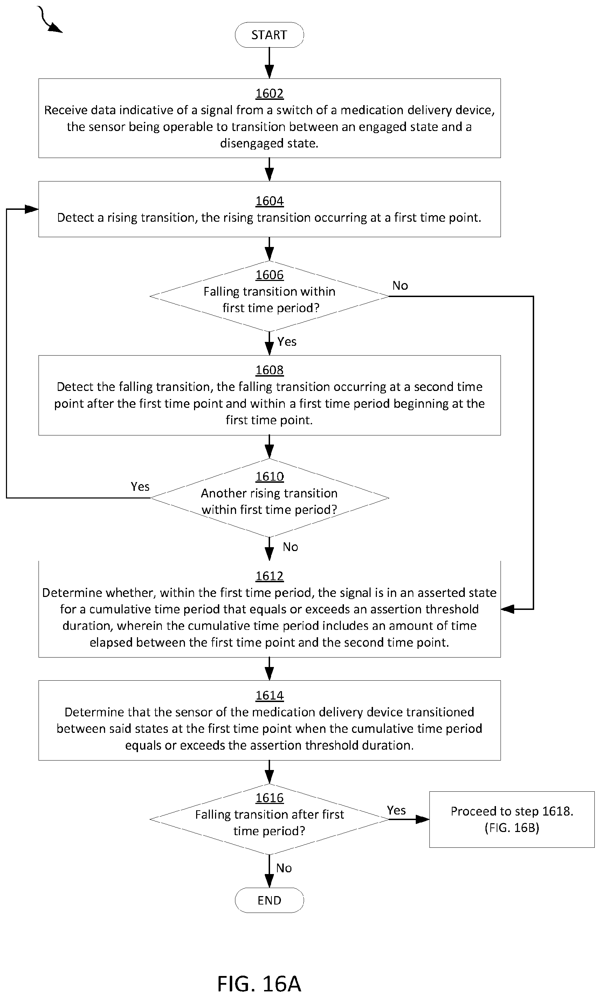

FIGS. 16A-16B is a flowchart showing an exemplary method 1600 for filtering a signal received from the sensor of a medication delivery device, according to some embodiments. Method 1600 may be implemented on any suitable processor, such as microcontroller 1204, microcontroller 1410, and/or one or more processors external to the medication delivery device, for example.

At step 1602, the processor receives data indicative of a signal from the sensor of the medication delivery device, such as the sensor 86 in FIGS. 5-7 and the sensor 1202 in FIG. 12, for example. In some embodiments, the processor receives the signal directly from the sensor 1202. For example, sensor 1202 may be placed in electrical communication with an input (e.g., a logic input) of the processor, such as a GPIO pin input of the processor. Additionally or alternatively, in some embodiments, the processor receives the signal indirectly from the sensor 1202. For example, the processor may receive the signal after it has been filtered using one or more components of the medication delivery device, such as an RC circuit (e.g., RC circuit 1424 in FIG. 14). Additionally or alternatively, the processor may receive data indicative of the signal, after the signal has been processed using any suitable pre-processing steps. Additionally or alternatively, the processor may receive data indicative of the signal from a different processor. For example, the processor may be external to the medication delivery device and receive the data indicative of the signal from a microcontroller included in the medication delivery device. In some embodiments, the raw or processed signal from sensor 1202 may be stored in memory for some time before the processor receives the data.

In some embodiments, the processor continues to receive the data indicative of the signal during subsequent steps of process 1600. For example, steps of process 1600 may be performed as the signal is being generated, and the processor may receive and process the data indicative of the newly generated portions of the signal at any time during process 1600.

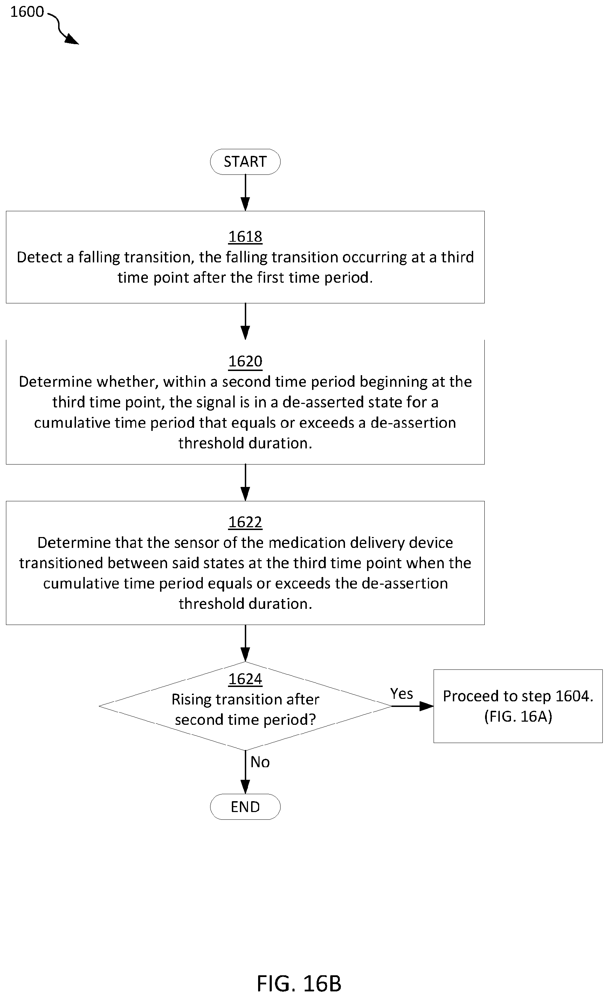

At step 1604, the processor detects, based on the received date, a transition of the signal to a first logic state (e.g., an asserted state) from a second logic state (e.g., a de-asserted state). For the sake of simplicity, the transition of a signal to a first logic state from a second logic state may be referred to herein as a "rising transition." It should be noted that the term "rising transition" is most appropriate for embodiments in which the first logic state corresponds to an asserted, or high logic state, and the second logic state corresponds to a de-asserted or low logic state, such that the signal "rises" from a low state to a high state when it transitions from the first logic state to the second logic state. However, as discussed previously, the present disclosure also contemplates embodiments in which the first logic state corresponds to a de-asserted or low logic state, and the second logic state corresponds to an asserted or high logic state. Use of the term "rising transition" herein as part of FIGs. 16A and 16B does not imply that such alternative embodiments are excluded.

In some embodiments, the processor is configured to determine the time point (e.g., a first time point) at which the rising transition occurred. The processor may determine the time point using any suitable technique. As an illustrative example, the processor may determine the time point using an interrupt handler implemented using software executing or configured to execute on the processor. In some embodiments, the interrupt handler is configured to log a timestamp indicating the time point at which the rising transition occurred. For example, the interrupt handler may log the timestamp according to a timer included in the medication delivery device. Additionally or alternatively, the processor may determine the time point by polling the timer included in the medication delivery device.

At step 1606, the processor determines whether a transition of the signal to the second logic state from the first logic state occurred within a first time period. For the sake of simplicity, the transition of a signal to the second logic state from the first logic state may be referred to herein as a "falling transition." It should again be noted that the term "falling transition" is most appropriate for embodiments in which the first logic state corresponds to an asserted or high logic state and the second logic state corresponds to a de-asserted or low logic state, such that the signal "falls" from a high state to a low state when it transitions from the first logic state to the second logic state. However, as discussed previously, the present disclosure also contemplates embodiments in which the first logic state corresponds to a de-asserted or low logic state and the second logic state corresponds to an asserted or high logic state. Use of the term "falling transition" herein as part of FIGs. 16A and 16B does not imply that such alternative embodiments are excluded.

In some embodiments, the first time period begins at the time point at which the rising transition occurred (e.g., the first time point), as determined at step 1604. In some embodiments, the duration of the first time period depends on the amount of time that the sensor is expected to be in an engaged state. For example, when the sensor is expected to be in an engaged state for up to one second, the duration of the first time period may be approximately one second (e.g., between 750ms and 1.25s, between 800ms and 1.2s, between 900ms and 1.1s, 1s, etc.). However, aspects of the technology are not limited in this respect, and the first time period may be of any suitable duration.

In some embodiments, if a falling transition is detected within the first time period, process 1600 proceeds to step 1608. If the falling transition is not detected within the first time period, process 1600 proceeds to step 1612.

At step 1608, the processor detects the falling transition of the signal based on the data received at step 1602 or data received at any time during process 1600. In some embodiments, the processor is configured to determine the time point (e.g., a second time point) at which the falling transition occurred. The processor may determine the time point using any suitable technique. For example, the processor may determine the time point using an interrupt handler implemented using software executing or configured to execute on the processor. In some embodiments, the interrupt handler is configured to log a timestamp indicating the time point at which the falling transition occurred. For example, the interrupt handler may log the timestamp according to a timer included in the medication delivery device. Additionally or alternatively, the processor may determine the time point by polling the timer included in the medication delivery device.

At step 1610, the processor determines whether another rising transition of the signal occurred within the first time period and after the second time point (e.g., the time at which the falling transition occurred). In some embodiments, if another rising transition occurred within the first time period, process 1600 returns to step 1604, where the rising transition is detected. If no additional rising transitions occurred within the first time period, process 1600 proceeds to step 1612.

At step 1612, the processor determines whether, within the first time period, the signal is in the first logic state for a cumulative time period that equals or exceeds a first threshold duration. In some embodiments, the signal is considered to be in the first logic state during the time elapsed between a rising transition of the signal and a falling transition of the signal. For example, the cumulative time period may include the amount of time elapsed between the first time point (e.g., determined at step 1604) and the second time point (e.g., determined at step 1608). If, at step 1610, there was another rising transition within the first time period then the cumulative time period may also include an amount of time elapsed between the time point at which the rising transition occurred and the time point of the occurrence of either: (a) the end of the first time period, or (b) the time point at which another falling transition occurred within the first time period. The logic described in the previous sentence may be applied to any further rising transitions in addition to the first and second rising transitions within the first time period. In some embodiments, if, at step 1606, no falling transition was determined to have occurred within the first time period, then the cumulative amount of time may include the amount of time elapsed between the first time point and the end of the first time period.

In some embodiments, the first threshold duration may include any suitable duration that is less than or equal to the duration of the first time period. As nonlimiting examples, the first threshold duration may be 70% of the duration of the first time period, 75% of the duration of the first time period, 80% of the duration of the first time period, 85% of the duration of the first time period, 90% of the duration of the first time, 100% of the duration of the first time period, or any other suitable threshold duration. In some embodiments, the first threshold duration may depend on the expected duration of sensor engagement and/or expected frequency of noise (e.g., momentary transitions of the signal to the second logic state) as the sensor engages with a sensed component (e.g., a tooth) of the medication delivery device. For example, a relatively low first threshold duration would allow for more noise when determining that the sensor is in an engaged state, as compared to a relatively high first threshold duration. In some embodiments, once the processor determines that the signal has been in the first logic state for a cumulative time period that equals the first threshold duration within the first time period, the processor may discontinue measuring the cumulative time period for which the signal is in the first logic state.

At step 1614, the processor determines whether the sensor transitioned from a disengaged state to an engaged state at the first time point. If the cumulative time period, determined at step 1612, equals or exceeds the first threshold duration, the processor determines that the sensor has transitioned from the disengaged state to the engaged state at the first time point. In some embodiments, the occurrence of such a transition may be included in a count indicating the number of times that the sensor transitioned between the disengaged state and the engaged state. In some embodiments, the count may be used to determine a dosage of medication delivered using the medication delivery device. If the cumulative time period does not equal or exceed the first threshold duration, the processor determines that the sensor has not transitioned from the disengaged state to the engaged state.

At step 1616, the processor determines whether a falling transition occurred after the first time period. If no falling transition occurred after the first time period, then process 1600 ends. If a falling transition did occur after the first time period, then process 1600 proceeds to step 1618, shown in FIG. 16B. Additionally, or alternatively, while not shown in FIG. 16A, if the cumulative time period equals or exceeds the first threshold duration, the processor can transition to step 1618 shown in FIG. 16B after the first threshold duration and/or after the first time period.

At step 1618, the processor detects the falling transition of the signal based on the data received at step 1602 or data received at any time during process 1600. In some embodiments, the processor is configured to determine the time point (e.g., a third time point) at which the falling transition occurred. The processor may determine the time point using any suitable technique. For example, the processor may determine the time point using an interrupt handler, such as the interrupt described herein including at least with respect to step 1608. Additionally or alternatively, the processor may determine the time point by polling the timer included in the medication delivery device.

At step 1620, the processor determines whether, within a second time period, the signal is in the second logic state for a cumulative time period that equals or exceeds a second threshold duration. In some embodiments, the second time period begins at the time point at which the falling transition occurred (e.g., the third time point), as determined at step 1618. In some embodiments, the duration of the second time period depends on the amount of time that the sensor is expected to be in a disengaged state. For example, when the sensor is expected to be in a disengaged state for up to one second, the duration of the second time period may be approximately one second (e.g., between 300ms and 1.7s, between 400ms and 1.6s, between 500ms and 1.5s, between 600ms and 1.4s, between 700ms and 1.3s, between 750ms and 1.25s, between 800ms and 1.2s, between 900ms and 1.1s, 1s, etc.). However, aspects of the technology are not limited in this respect, and the second time period may be of any suitable duration.

In some embodiments, the signal is considered to be in the second logic state during the time elapsed between a falling transition of the signal and a rising transition of the signal. For example, the cumulative time period may include the amount of time elapsed between the third time point (e.g., determined at step 1618) and a later time point (e.g.., a fourth time point), during the second time period, at which a rising transition of the signal occurred. If there is another falling transition after the fourth time point and within the second time period, then the cumulative time period may also include an amount of time elapsed between the time point at which the falling transition occurred and the time point of the occurrence of either: (a) the end of the second time period, or (b) the time point at which another rising transition occurred within the second time period. The logic described in the previous sentence may be applied to any further falling transitions in addition to the first and second falling transitions within the second time period. In some embodiments, if no rising transition occurred after the third time point and within the second time period, then the cumulative time period may include the amount of time elapsed between the third time point and the end of the second time period.

In some embodiments, the second threshold duration may be any suitable duration that is less than or equal to the duration of the second time period. As nonlimiting examples, the second threshold duration may be 70% of the duration of the second time period, 75% of the duration of the second time period, 80% of the duration of the second time period, 85% of the duration of the second time period, 90% of the duration of the first time, 100% of the duration of the second time period, or any other suitable second threshold duration. In some embodiments, the second threshold duration may depend on the expected duration of sensor disengagement and/or the expected frequency of noise (e.g., momentary signal transitions to the first logic state) expected as the sensor disengages from a sensed component (e.g., a tooth) of the medication delivery device. The second threshold duration may be the same as or different from the first threshold duration.

At step 1622, the processor determines whether the sensor of the medication delivery device transitioned between the engaged state and the disengaged state at the third time point. If the cumulative time period, determined at step 1620, equals or exceeds the second threshold duration, then the processor determines that the medication delivery device transitioned between the engaged state and the disengaged state. If the cumulative time period does not equal or exceed the second threshold duration, then the processor determines that the medication delivery device did not transition between the engaged state and the disengaged state.

At step 1624, the processor determines whether another rising transition of the signal occurred after the second time period. If a rising transition did occur, then process 1600 returns to step 1604, shown in FIG. 16A. If a rising transition did not occur, then process 1600 ends.

For the sake of simplicity, FIGs. 16A and 16B depict an exemplary process for determining whether the sensor transitioned from said disengaged state to said engaged state (or vice versa) in which the first logic state corresponds to an asserted or high logic state and in which the second logic state corresponds to a de-asserted or low logic state. This does not imply that the present disclosure excludes embodiments in which the first logic state corresponds instead to a de-asserted or low logic state and in which the second logic state corresponds instead to an asserted or high logic state. In such alternative embodiments, appropriate modifications may be made to FIGs. 16A and 16B. For instance, the term "rising transition" may be replaced with the term "falling transition" (and vice versa). Similarly, the term "asserted state" or "assertion threshold duration" may be replaced with the term "de-asserted state" or "de-assertion threshold duration" (and vice versa).

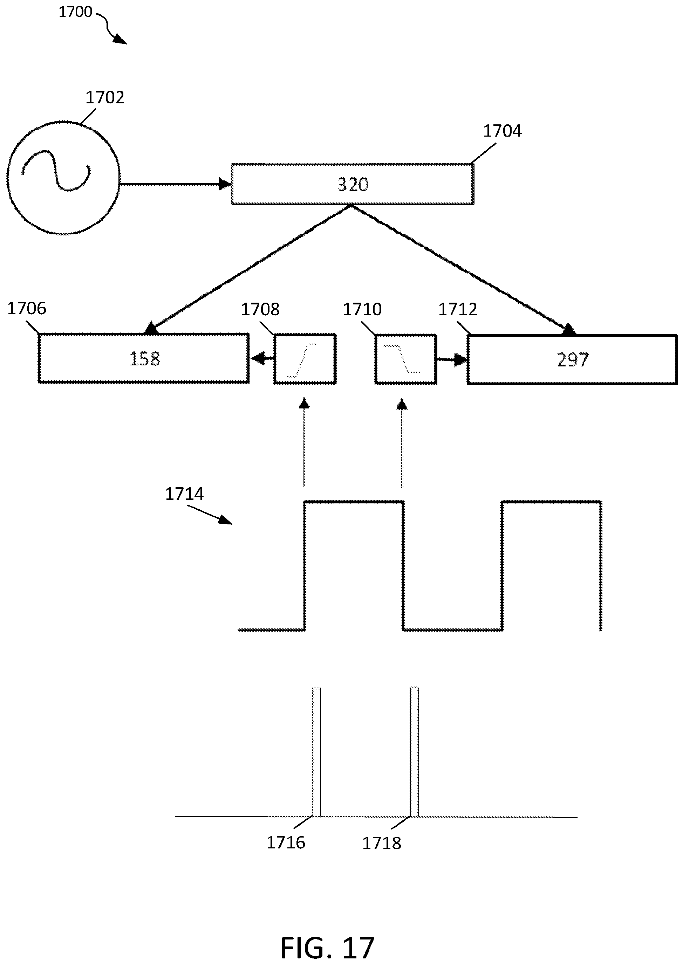

FIG. 17 is an exemplary schematic diagram showing a system 1700 for detecting rising and falling transitions of a signal received from the sensor of a medication delivery device, according to some embodiments. As shown, system 1700 includes clock 1702, timer 1704, rising transition interrupt handler 1716, and falling transition interrupt handler 1718. It should be appreciated, however, that a system for detecting rising and/or falling transitions may include one or more additional or alternative components, as aspects of the technology described herein are not limited in this respect.

In some embodiments, the rising transition interrupt handler 1716 is configured to detect a rising transition 1708 of signal 1714 and log a timestamp 1706 indicating the time point at which the rising transition 1708 occurred. For example, timestamp 1706 indicates that rising transition 1708 occurred at count 158, according to timer 1704.

In some embodiments, the rising transition interrupt handler 1716 is an interrupt configured to detect a rising transition 1708 of a signal 1714 and/or log a time stamp at which the rising transition 1708 occurred. The rising transition interrupt handler 1716 may be implemented using software executing or configured to execute on a processor such as the processor configured perform process 1600 shown in FIGS. 16A-16B, for example.

In some embodiments, the timer 1704 is any suitable timer such as, for example, a timer configured to count up from an initial time. For example, clock 1702 may cause timer 1704 to count up from a time that a medication delivery device is assembled. In some embodiments, the timer 1704 may be configured to count up at some rate (e.g., in one second increments) to a particular value, then start over when it reaches that value. For example, the clock 1702 may drive an 11-bit counter to count up, in one second increments, to 2,047. When the counter achieves 2,047, it re-starts at 0. In some embodiments, in order to keep track of the cumulative time elapsed since the initial time, system 1700 is configured to log each time the counter re-starts. In some embodiments timer 1704 is included in a medication delivery device. For example, the timer may be included on a printed circuit board (PCB) of a medication delivery device. In some embodiments, the timer 1704 is external to the medication delivery device.

In some embodiments, the clock 1702 includes any suitable clock such as, for example, system clock 1430 shown in FIG. 14. In some embodiments, clock 1702 is included in a medication delivery device. For example, the clock 1702 may be included on a PCB of a medication delivery device. In some embodiments, clock 1702 is external to the medication delivery device.

In some embodiments, the falling transition interrupt handler 1718 is configured to detect a falling transition 1710 of signal 1714 and log a timestamp 1712 indicating the time point at which the falling transition 1710 occurred. For example, timestamp 1712 indicates that falling transition 1710 occurred at count 297, according to timer 1704.

In some embodiments, the falling transition interrupt handler 1718 is an interrupt configured to detect a falling transition 1710 of a signal 1714 and/or log a time stamp at which the falling transition 1710 occurred. The falling transition interrupt handler 1718 may be implemented using software executing or configured to execute on a processor such as the processor configured perform process 1600 shown in FIGS. 16A-16B, for example.

In some embodiments, the timestamps 1706, 1712 that are logged by interrupt handlers 1716, 1718 can be used to determine durations of time during which the signal 1714 was in a particular state. In the example of FIG. 17, the signal 1714 is in an asserted state for a duration defined by the difference between the time point indicated by timestamp 1706 and the time point indicated by timestamp 1712. As shown, the signal 1714 is in an asserted state for a count of 139, which is equal to the difference between 297 and 158. If the timer 1704 is configured to count up in one microsecond increments, then the duration of time during which the signal is in the asserted state is 139 microseconds. In some embodiments, if the timer 1704 re-starts its count (e.g., restarts at 0) at some time between the occurrence of the rising transition 1708 and the falling transition 1710, then additional information may be used to determine an amount of time elapsed between the two occurrences. Such additional information may include, for example, the maximum value that the timer 1704 is configured to count up to and/or the number of times that the timer 1704 has restarted its count.

FIG. 18 is an exemplary plot showing an integration of a signal received from the sensor of a medication delivery device, according to some embodiments. As shown, line 1810 indicates the times at which a sensor of a medication delivery device actually transitioned between an engaged state and a disengaged state. Line 1820 indicates the signal received from a sensor of the medication delivery device. Line 1830 indicates the cumulative time period that the signal 1820 was in an asserted state and/or a de-asserted state.

In some embodiments, the signal 1820 is received by a processor. In some embodiments, the processor is configured to process the signal by performing part or all of process 1600 described herein including at least with respect to FIGS. 16A-16B.

For example, in some embodiments, the processor may detect or be configured to detect a first rising transition of the signal 1820. In detecting the first rising transition, the processor may determine a first time point at which the first rising transition occurred. For example, the processor may use a software-implemented interrupt handler to log a timestamp indicating the first time point at which the first rising transition occurred. Additionally or alternatively, in some embodiments, the first time point may not correspond to the first rising transition. For example, the first time point may include any suitable time point determined in any suitable manner, such as a time point before or after the occurrence of the first rising transition, as aspects of the technology described herein are not limited in this respect. For example, the first time period may essentially slide along upon detection of each rising transition of the signal to search for the first instance of a series of signals that result in the signal being in the asserted state for the first threshold duration (e.g., an assertion threshold duration).

In some embodiments, the first time point marks the beginning of the first time period, as shown in FIG. 18. The processor may further detect or be configured to detect additional rising and/or falling transitions that occurred within the first time period, after the first time point. This may include, for example, determining a time point at which each rising and/or falling transition occurred during the first time period, such as a second time point at which a first falling transition occurred. As shown, three additional rising transitions and three falling transitions of the signal 1820 occurred within the first time period.

In some embodiments, the processor may determine or be configured to determine whether the sensor transitioned between the disengaged state and the engaged state at the first time point. In some embodiments, this includes determining whether the sensor was in an asserted state for a cumulative time period, within the first time period, that equals or exceeds the assertion threshold duration (T_{A}).

In some embodiments, determining the cumulative time period includes (a) determining an amount of time elapsed between each rising and falling transition within the first time period, and (b) summing the determined amounts of time. For example, line 1830 shows an integration over the signal 1820 in the first time period. Between each rising transition and falling transition within the first time period, when the signal 1820 is in an asserted state, the line 1830 rises by the amount of time elapsed during the signal assertion. In other words, the amount of time elapsed is included in the cumulative time period. However, between each falling transition and rising transition, when the signal 1820 is in a de-asserted state, the line 1830 does not increase. In other words, the amount of time elapsed during a signal de-assertion is not included in the cumulative time period. In some embodiments, when there is a rising transition that is not following by a falling transition within the first time period, as shown by the final rising transition in the first time period in FIG. 18, the cumulative time period includes the time elapsed between the time point at which the rising transition occurred and the time point at which the first time period ends.

In some embodiments, if the cumulative time period equals or exceeds the assertion threshold duration (T_{A}), then the processor determines that the sensor transitioned to an engaged state at the first time point. As shown in the example of FIG. 18, within the first time period, the cumulative time period indicated by line 1830 equals the assertion threshold duration (T_{A}). Therefore, even though there was noise (e.g., momentary signal de-assertions) within the first time period, the processor still correctly determines that the sensor transitioned to the engaged state at the first time point

In some embodiments, after determining that the sensor transitioned to an engaged state at the first time point, the processor may evaluate the quality of the signal 1820. In some embodiments, such an evaluation may include determining the number of rising and/or falling transitions of the signal within the first time period. For example, during the first time period shown in FIG. 18, there were four rising transitions (including the first rising transition) and three falling transitions. In some embodiments, if the number of rising and/or falling transitions equals or exceeds a specified threshold (e.g., the signal is very noisy), this may indicate that there may be an issue with the sensor and therefore, there may be a lower confidence associated with determining that the sensor transitioned to an engaged state. In some embodiments, when this occurs, even when the processor previously determined that the sensor transitioned to an engaged state (e.g., before the quality evaluation), the processor may discount that transition.

In some embodiments, the processor may provide output indicative of the quality of the signal. For example, when the number of rising and/or falling transitions exceeds the threshold, the processor may provide output recommending that a user manually check the dosage administered by the medication delivery device to confirm the accuracy of the estimate made based on the signal. Additionally or alternatively, the processor may provide output as part of a quality control process. For example, during manufacturing, the processor may output a metric indicative of the number of rising and/or falling transitions in the signal, which may then be compared to an expected value. If there is a discrepancy between these two values, then the tested medication delivery device and/or manufacturing process may be flagged for potential issues.

In some embodiments, the processor may further detect or be configured to detect a falling transition of the signal 1820 that occurred after the first time period. In the example of FIG. 18, a falling transition occurred at the third time point, after the first time period. In some embodiments, in detecting the falling transition, the processor may determine the third time point at which the falling transition occurred. For example, the processor may use a software-implemented interrupt handler to log a timestamp indicating the third time point at which the falling transition occurred. Additionally or alternatively, in some embodiments, the third time point may not correspond to the falling transition. For example, the third time point may correspond to the time at which the first time period ended. For example, the second time period may essentially slide along in time upon detection of each falling edge to search for the first instance of a series of signals that result in the signal being in the de-asserted state for the second threshold duration (e.g., de-assertion threshold duration). As described above, the first time period can additionally or alternatively slide along in time to perform the detection as described herein.

In some embodiments, the third time point marks the beginning of the second time period, as shown in FIG. 18. The processor may further detect or be configured to detect additional falling and/or rising transitions that occurred within the second time period, after the third time point. This may include, for example, determining a time point at which each falling and/or rising transition occurred during the second time period. As indicated, two additional falling transitions and two rising transitions of the signal 1820 occurred within the second time period.

In some embodiments, the processor may determine or be configured to determine whether the sensor transitioned between the engaged state and the disengaged state at the third time point. In some embodiments, this includes determining whether the sensor was in a de-asserted state for a cumulative time period that equals or exceeds the de-assertion threshold duration (T_{D}).

In some embodiments, determining the cumulative time period includes (a) determining an amount of time elapsed between each falling and rising transition within the second time period, and (b) summing the determined amounts of time. For example, line 1830 shows an integration over the signal 1820 during the second time period. Between each falling transition and rising transition within the second time period, when the signal 1820 is in a de-asserted state, the line 1830 rises by the amount of time elapsed during the signal de-assertion. In other words, the amount of time elapsed is included in the cumulative time period. However, between each rising transition and falling transition, when the signal 1820 is in an asserted state, the line 1830 does not increase. In other words, the amount of time elapsed during a signal assertion is not included in the cumulative time period. In some embodiments, when there is a falling transition that is not followed by a rising transition within the second time period, as shown by the final falling transition in the second time period in FIG. 18, the cumulative time period includes the time elapsed between the time point at which the falling transition occurred and the time point at which the second time period ends.

In some embodiments, if the cumulative time period equals or exceeds the de-assertion threshold duration (T_{D}), then the processor determines that the sensor transitioned to a disengaged state at the third time point. As shown in the example of FIG. 18, within the second time period, the cumulative time period indicated by line 1830 equals the de-assertion threshold duration (T_{D}). Therefore, even though there was noise (e.g., momentary signal assertions) within the second time period, the processor still correctly determines that the sensor transitioned to the disengaged state at the third time point.

In some embodiments, the techniques can be configured to process the signal as the signal is being received (e.g., in real-time during signal receipt and/or processing). Such an approach can, for example, achieve memory savings since the entire signal need not be saved for the medication delivery device. In some embodiments, the techniques can be configured to store the received signal and to process the stored signal at a later point in time (i.e., not in real-time during signal receipt and/or processing) according to the techniques described herein. While such techniques may require more memory compared to processing the signal at receipt, such techniques can allow for the signal to processed in multiple passes, etc., such that the signal can be processed various ways to determine the best way(s) to analyze the signal to determine assert and de-assert states.

It should be appreciated that various time periods, such as the first and/or second time periods, are configurable and may change over time as the signal is processed. In some embodiments, the first time period is different than the second time period. In some embodiments, the first time period is the same as the second time period. In some embodiments, multiple different first time periods and/or second time periods can be used in accordance with the techniques described herein.

While FIG. 18 has been depicted in the context of embodiments in which the first logic state corresponds to an asserted or high logic state and in which the second logic state corresponds to a de-asserted or low logic state, other embodiments are also possible in which the first logic state corresponds to a de-asserted state and in which the second logic state corresponds to a de-asserted state. In such alternative embodiments, appropriate modifications may be made to FIG. 18. For example, line 1820 for the signal may be inverted such that every time line 1810 indicates the sensor is in an engaged state, the signal 1820 occupies a de-asserted state, and similarly, every time line 1810 indicates the sensor is in a dis-engaged state, the signal 1820 occupies an asserted state. The Assertion Threshold Duration (T_{A}) may be modified to become a De-assertion Threshold Duration (T_{D}). Within the first time period, the cumulative time period indicated by line 1830 may correspond to a cumulative amount of time that the signal 1820 spends in a de-asserted state instead of an asserted state. The De-Assertion Threshold Duration (T_{D}) may be modified to become an Assertion Threshold Duration (T_{A}). Within the second time period, the cumulative time period indicated by line 1830 may correspond to a cumulative amount of time that the signal 1820 spends in an asserted state instead of a de-asserted state.

In some embodiments, the processor may determine a quality metric based on the processing of the signal 1820. The metric may include any suitable metric such as, for example, the ratio between the number of times that the sensor was determined to have transitioned to an engaged state and the number of rising transitions in the signal. Additionally or alternatively, the metric may include the ratio between the number of times that the sensor transitioned to a disengaged state and the number of falling transitions in the signal.

In some embodiments, the quality metric may be output to a user, such as a user of the medication delivery device, a healthcare provider, and/or a user involved in manufacturing and/or testing the medication delivery device, for example. In some embodiments, the quality metric may indicate that there is an issue with the sensor of the medication delivery device, such as, for example, when the ratio between signal transitions and sensor transitions is large (e.g., indicating that the signal is noisy). Additionally or alternatively, when the quality metric is indicative of an issue, a recommendation may be output to the user to check the sensor, to manually check the dosage delivered using the medication delivery device, and/or any other suitable recommendation, as aspects of the technology are not limited in this respect.

The device described herein is a reusable pen-shaped medication injection device, generally designated, which is manually handled by a user to selectively set a dose and then to inject that set dose. Injection devices of this type are well known, and the description of device is merely illustrative as the sensing system can be adapted for use in variously configured medication delivery devices, including differently constructed pen-shaped medication injection devices, differently shaped injection devices, and infusion pump devices. The medication may be any of a type that may be delivered by such a medication delivery device. Device is intended to be illustrative and not limiting as the sensing system described further below may be used in other differently configured devices.

Techniques operating according to the principles described herein may be implemented in any suitable manner. The processing and decision blocks of the flow charts above represent steps and acts that may be included in algorithms that carry out these various processes. Algorithms derived from these processes may be implemented as software integrated with and directing the operation of one or more single- or multi-purpose processors, may be implemented as functionally-equivalent circuits such as a Digital Signal Processing (DSP) circuit or an Application-Specific Integrated Circuit (ASIC), or may be implemented in any other suitable manner. It should be appreciated that the flow charts included herein do not depict the syntax or operation of any particular circuit or of any particular programming language or type of programming language. Rather, the flow charts illustrate the functional information one skilled in the art may use to fabricate circuits or to implement computer software algorithms to perform the processing of a particular apparatus carrying out the types of techniques described herein. It should also be appreciated that, unless otherwise indicated herein, the particular sequence of steps and/or acts described in each flow chart is merely illustrative of the algorithms that may be implemented and can be varied in implementations and embodiments of the principles described herein.

Accordingly, in some embodiments, the techniques described herein may be embodied in computer-executable instructions implemented as software, including as application software, system software, firmware, middleware, embedded code, or any other suitable type of computer code. Such computer-executable instructions may be written using any of a number of suitable programming languages and/or programming or scripting tools, and also may be compiled as executable machine language code or intermediate code that is executed on a framework or virtual machine.

When techniques described herein are embodied as computer-executable instructions, these computer-executable instructions may be implemented in any suitable manner, including as a number of functional facilities, each providing one or more operations to complete execution of algorithms operating according to these techniques. A "functional facility," however instantiated, is a structural component of a computer system that, when integrated with and executed by one or more computers, causes the one or more computers to perform a specific operational role. A functional facility may be a portion of or an entire software element. For example, a functional facility may be implemented as a function of a process, or as a discrete process, or as any other suitable unit of processing. If techniques described herein are implemented as multiple functional facilities, each functional facility may be implemented in its own way; all need not be implemented the same way. Additionally, these functional facilities may be executed in parallel and/or serially, as appropriate, and may pass information between one another using a shared memory on the computer(s) on which they are executing, using a message passing protocol, or in any other suitable way.

Generally, functional facilities include routines, programs, objects, components, data structures, etc. that perform particular tasks or implement particular abstract data types. Typically, the functionality of the functional facilities may be combined or distributed as desired in the systems in which they operate. In some implementations, one or more functional facilities carrying out techniques herein may together form a complete software package. These functional facilities may, in alternative embodiments, be adapted to interact with other, unrelated functional facilities and/or processes, to implement a software program application.

Some exemplary functional facilities have been described herein for carrying out one or more tasks. It should be appreciated, though, that the functional facilities and division of tasks described is merely illustrative of the type of functional facilities that may implement the exemplary techniques described herein, and that embodiments are not limited to being implemented in any specific number, division, or type of functional facilities. In some implementations, all functionality may be implemented in a single functional facility. It should also be appreciated that, in some implementations, some of the functional facilities described herein may be implemented together with or separately from others (i.e., as a single unit or separate units), or some of these functional facilities may not be implemented.

Computer-executable instructions implementing the techniques described herein (when implemented as one or more functional facilities or in any other manner) may, in some embodiments, be encoded on one or more computer-readable media to provide functionality to the media. Computer-readable media include magnetic media such as a hard disk drive, optical media such as a Compact Disk (CD) or a Digital Versatile Disk (DVD), a persistent or non-persistent solid-state memory (e.g., Flash memory, Magnetic RAM, etc.), or any other suitable storage media. Such a computer-readable medium may be implemented in any suitable manner. As used herein, "computer-readable media" (also called "computer-readable storage media") refers to tangible storage media. Tangible storage media are non-transitory and have at least one physical, structural component. In a "computer-readable medium," as used herein, at least one physical, structural component has at least one physical property that may be altered in some way during a process of creating the medium with embedded information, a process of recording information thereon, or any other process of encoding the medium with information. For example, a magnetization state of a portion of a physical structure of a computer-readable medium may be altered during a recording process.

Further, some techniques described above comprise acts of storing information (e.g., data and/or instructions) in certain ways for use by these techniques. In some implementations of these techniques-such as implementations where the techniques are implemented as computer-executable instructions-the information may be encoded on a computer-readable storage media. Where specific structures are described herein as advantageous formats in which to store this information, these structures may be used to impart a physical organization of the information when encoded on the storage medium. These advantageous structures may then provide functionality to the storage medium by affecting operations of one or more processors interacting with the information; for example, by increasing the efficiency of computer operations performed by the processor(s).

In some, but not all, implementations in which the techniques may be embodied as computer-executable instructions, these instructions may be executed on one or more suitable computing device(s) operating in any suitable computer system, or one or more computing devices (or one or more processors of one or more computing devices) may be programmed to execute the computer-executable instructions. A computing device or processor may be programmed to execute instructions when the instructions are stored in a manner accessible to the computing device or processor, such as in a data store (e.g., an on-chip cache or instruction register, a computer-readable storage medium accessible via a bus, a computer-readable storage medium accessible via one or more networks and accessible by the device/processor, etc.). Functional facilities comprising these computer-executable instructions may be integrated with and direct the operation of a single multi-purpose programmable digital computing device, a coordinated system of two or more multi-purpose computing device sharing processing power and jointly carrying out the techniques described herein, a single computing device or coordinated system of computing device (co-located or geographically distributed) dedicated to executing the techniques described herein, one or more Field-Programmable Gate Arrays (FPGAs) for carrying out the techniques described herein, or any other suitable system.

A computing device may comprise at least one processor, a network adapter, and computer-readable storage media. A computing device may be, for example, a desktop or laptop personal computer, a personal digital assistant (PDA), a smart mobile phone, a server, or any other suitable computing device. A network adapter may be any suitable hardware and/or software to enable the computing device to communicate wired and/or wirelessly with any other suitable computing device over any suitable computing network. The computing network may include wireless access points, switches, routers, gateways, and/or other networking equipment as well as any suitable wired and/or wireless communication medium or media for exchanging data between two or more computers, including the Internet. Computer-readable media may be adapted to store data to be processed and/or instructions to be executed by processor. The processor enables processing of data and execution of instructions. The data and instructions may be stored on the computer-readable storage media.

A computing device may additionally have one or more components and peripherals, including input and output devices. These devices can be used, among other things, to present a user interface. Examples of output devices that can be used to provide a user interface include printers or display screens for visual presentation of output and speakers or other sound generating devices for audible presentation of output. Examples of input devices that can be used for a user interface include keyboards, and pointing devices, such as mice, touch pads, and digitizing tablets. As another example, a computing device may receive input information through speech recognition or in other audible format.

Embodiments have been described where the techniques are implemented in circuitry and/or computer-executable instructions. It should be appreciated that some embodiments may be in the form of a method, of which at least one example has been provided. The acts performed as part of the method may be ordered in any suitable way. Accordingly, embodiments may be constructed in which acts are performed in an order different than illustrated, which may include performing some acts simultaneously, even though shown as sequential acts in illustrative embodiments.

Various aspects of the embodiments described above may be used alone, in combination, or in a variety of arrangements not specifically discussed in the embodiments described in the foregoing and is therefore not limited in its application to the details and arrangement of components set forth in the foregoing description or illustrated in the drawings. For example, aspects described in one embodiment may be combined in any manner with aspects described in other embodiments.

Use of ordinal terms such as "first," "second," "third," etc., in the claims to modify a claim element does not by itself connote any priority, precedence, or order of one claim element over another or the temporal order in which acts of a method are performed, but are used merely as labels to distinguish one claim element having a certain name from another element having a same name (but for use of the ordinal term) to distinguish the claim elements.

Also, the phraseology and terminology used herein is for the purpose of description and should not be regarded as limiting. The use of "including," "comprising," "having," "containing," "involving," and variations thereof herein, is meant to encompass the items listed thereafter and equivalents thereof as well as additional items.

The word "exemplary" is used herein to mean serving as an example, instance, or illustration. Any embodiment, implementation, process, feature, etc. described herein as exemplary should therefore be understood to be an illustrative example and should not be understood to be a preferred or advantageous example unless otherwise indicated.

To clarify the use of and to hereby provide notice to the public, the phrases "at least one of <A>, <B>, ... and <N>" or "at least one of <A>, <B>, . . . <N>, or combinations thereof" or "<A>, <B>, . . . and/or <N>" are defined by the Applicant in the broadest sense, superseding any other implied definitions hereinbefore or hereinafter unless expressly asserted by the Applicant to the contrary, to mean one or more elements selected from the group comprising A, B, . . . and N. In other words, the phrases mean any combination of one or more of the elements A, B, . . . or N including any one element alone or the one element in combination with one or more of the other elements which may also include, in combination, additional elements not listed.

While various embodiments have been described, it will be apparent to those of ordinary skill in the art that many more embodiments and implementations are possible. Accordingly, the embodiments described herein are examples, not the only possible embodiments and implementations. Furthermore, the advantages described above are not necessarily the only advantages, and it is not necessarily expected that all of the described advantages will be achieved with every embodiment.

## Claims

1. A method for filtering a signal output from a sensor (86, 1202) of a medication delivery device (10), wherein the sensor is operable to transition between an engaged state in which the signal output from the sensor is in a first logic state and a disengaged state in which the signal output from the sensor is in a second logic state, the method comprising:
detecting a first transition of the signal to the first logic state from the second logic state, the first transition occurring at a first time point, and being **characterized by** further comprising:
determining whether, within a first time period beginning at the first time point, the signal is in the first logic state for a cumulative time period that equals or exceeds a first threshold duration; and
determining that the sensor of the medication delivery device transitioned from said disengaged state to said engaged state at the first time point when the cumulative time period equals or exceeds the first threshold duration.

2. The method of claim 1, further comprising determining that the sensor (86, 1202) of the medication delivery device (10) did not transition from said disengaged state to said engaged state when the cumulative time period does not equal or exceed the first threshold duration.

3. The method of any of claims 1-2,
further comprising detecting a second transition of the signal to the second logic state from the first logic state, the second transition occurring at a second time point after the first time point and within the first time period,
wherein the cumulative time period includes a first amount of time elapsed between the first time point and the second time point, wherein, optionally:
the method further comprises: detecting a third transition of the signal to the first logic state from the second logic state, the third transition occurring at a third time point after the second time point and within the first time period; and detecting a fourth transition of the signal to the second logic state from the first logic state, the fourth transition occurring at a fourth time point after the third time point and also within the first time period, wherein the cumulative time period includes a second amount of time elapsed between the third time point and the fourth time point but excludes an amount of time elapsed between the second time point and the third time point; and/or
detecting the second transition comprises logging the second time point at which the second transition occurred using a first interrupt handler.

4. The method of any of claims 1-3, wherein detecting the first transition comprises logging the first time point at which the first transition occurred using a second interrupt handler.

5. The method of any of claims 1-4, wherein determining that the sensor (86, 1202) of the medication delivery device (10) transitioned between said states further comprises:
determining whether a number of signal transitions occurring within the first time period exceeds a threshold; and
determining that the sensor of the medication delivery device transitioned from said disengaged state to said engaged state only when the number of signal transitions does not exceed the threshold.

6. The method of any of claims 1-5, further comprising:
detecting a fifth transition of the signal to the second logic state from the first logic state, the fifth transition occurring at a fifth time point after the first time period.

7. The method of claim 6, further comprising:
determining whether, within a second time period beginning at the fifth time point, the signal is in the second logic state for a second cumulative time period that equals or exceeds a second threshold duration; and
determining that the sensor (86, 1202) of the medication delivery device (10) transitioned from said engaged state to said disengaged state at the fifth time point when the second cumulative time period equals or exceeds the second threshold duration, wherein, optionally:
the method further comprises detecting a sixth transition of the signal to the first logic state from the second logic state, the sixth transition occurring at a sixth time point after the fifth time point and within the second time period, wherein the second cumulative time period includes an amount of time elapsed between the fifth time point and the sixth time point; and/or
a first duration of the first time period is different from a second duration of the second time period; or the first threshold duration is different from the second threshold duration; or both.

8. The method of any of claims 1-7, further comprising:
detecting a seventh transition to the first logic state from the second logic state, the seventh transition occurring at a seventh time point;
determining whether, within a third time period beginning at the seventh time point, the signal is in the first logic state for a third cumulative time period that equals or exceeds the first threshold duration; and
determining that the sensor (86, 1202) of the medication delivery device (10) transitioned from said disengaged state to said engaged state at the seventh time point when the third cumulative time period equals or exceeds the first threshold duration.

9. The method of any of claims 1-8, further comprising:
determining a number of sensor transitions occurring within a fourth time period beginning at the first time point, wherein the number of sensor transitions is indicative of an amount of medication delivered using the medication delivery device (10).

10. The method of any of claims 1-9, wherein:
the first logic state comprises an asserted state; and
the second logic state comprises a de-asserted state.

11. A non-transitory computer-readable storage media comprising instructions that, when executed by one or more processors on a computing device, are operable to cause the one or more processors to execute the method of any of claims 1-10.

12. A medication delivery device (10) comprising:
a housing (12) comprising a reservoir sized sufficiently to hold medication;
a printed circuit board (77, 1200, 1400);
a sensor (86, 1202) mounted to the printed circuit board and operable to output a signal, wherein the sensor is operable to transition between an engaged state in which the signal output from the sensor is in a first logic state and a disengaged state in which the signal output from the sensor is in a second logic state; and
a microcontroller (1204, 1410) in electrical communication with the sensor through a logic input to the microcontroller, wherein the microcontroller is configured to receive the signal output from the sensor,
**characterized in that** the microcontroller is further configured to determine, based on the received signal, whether the sensor has transitioned between said disengaged state and said engaged state at least in part by:
determining whether, within a first time period beginning at a first time point corresponding to a first transition of the signal to the first logic state from the second logic state, the signal is in the first logic state for a cumulative time period that equals or exceeds a first threshold duration; and
determining that the sensor has transitioned from said disengaged state to said engaged state when the cumulative time period equals or exceeds the first threshold duration.

13. The medication delivery device (10) of claim 12, wherein the microcontroller (1204, 1410) is configured to determine that the sensor (86, 1202) has not transitioned from said disengaged state to said engaged state when the cumulative time period does not equal or exceed the first threshold duration.

14. The medication delivery device (10) of any of claims 12-13, further comprising a rotatable element (38) that is rotatable relative to the printed circuit board (77, 1200, 1400), the rotatable element having a series of projections (102) that are spaced from one another, the rotatable element being positioned to permit the projections to slide against the sensor (86, 1202) to move the sensor between said engaged state and said disengaged state as the rotatable element rotates.

15. The medication delivery device (10) of any of claims 12-14, further comprising a resistor-capacitor (RC) circuit (1424) electrically coupled to the sensor (86, 1202) and the microcontroller (1204, 1410), wherein the signal is a filtered signal, and wherein the RC circuit is configured to:
receive an unfiltered signal from the sensor; and
transmit the filtered signal to the microcontroller.

16. The medication delivery device (10) of any of claims 12-14, further comprising a timer (1704) configured to count up from an initial time point, wherein, optionally, determining whether the sensor (86, 1202) has transitioned between said disengaged state and said engaged state further comprises:
detecting the first transition of the signal; and determining, using the timer, the first time point at which the first transition occurred; and/or
detecting a second transition of the signal to the second logic state from the first logic state, the second transition occurring at a second time point after the first time point and within the first time period; determining, using the timer, the second time point at which the second transition occurred; and determining an amount of time elapsed between the first time point and the second time point.

17. The medication delivery device of any of claims 12-16, further comprising medication held within the reservoir, optionally wherein the medication is insulin.

## Patentansprüche

1. Verfahren zum Filtern eines Signals, das von einem Sensor (86, 1202) einer Medikamentenabgabevorrichtung (10) ausgegeben wird, wobei der Sensor betriebsfähig ist, um zwischen einem in Eingriff stehenden Zustand, in dem sich das Signal, das von dem Sensor ausgegeben wird, in einem ersten Logikzustand befindet, und einem außer Eingriff stehenden Zustand zu wechseln, in dem sich das Signal, das von dem Sensor ausgegeben wird, in einem zweiten Logikzustand befindet, das Verfahren umfassend:
Erfassen eines ersten Übergangs des Signals in den ersten Logikzustand aus dem zweiten Logikzustand, wobei der erste Übergang zu einem ersten Zeitpunkt auftritt, und **gekennzeichnet ist durch** ferner umfassend:
Bestimmen, ob sich, innerhalb einer ersten Zeitspanne, die zu dem ersten Zeitpunkt beginnt, das Signal für eine kumulative Zeitspanne, die einer ersten Schwellenwertdauer gleicht oder diese überschreitet, in dem ersten Logikzustand befindet; und
Bestimmen, dass der Sensor der Medikamentenabgabevorrichtung zu dem ersten Zeitpunkt aus dem außer Eingriff stehenden Zustand in den in Eingriff stehenden Zustand übergegangen ist, wenn die kumulative Zeitspanne gleich der ersten Schwellenwertdauer ist oder diese überschreitet.

2. Verfahren nach Anspruch 1, ferner umfassend das Bestimmen, dass der Sensor (86, 1202) der Medikamentenabgabevorrichtung (10) nicht von dem außer Eingriff stehenden Zustand in den in Eingriff stehenden Zustand übergegangen ist, wenn die kumulative Zeitspanne nicht gleich der ersten Schwellenwertdauer ist oder diese überschreitet.

3. Verfahren nach einem der Ansprüche 1 bis 2,
ferner umfassend das Erfassen eines zweiten Übergangs des Signals in den zweiten Logikzustand aus dem ersten Logikzustand, wobei der zweite Übergang zu einem zweiten Zeitpunkt nach dem ersten Zeitpunkt und innerhalb der ersten Zeitspanne auftritt,
wobei die kumulative Zeitspanne eine erste Zeitmenge einschließt, die zwischen dem ersten Zeitpunkt und dem zweiten Zeitpunkt verstrichen ist, wobei, optional:
das Verfahren ferner umfasst: Erfassen eines dritten Übergangs des Signals in den ersten Logikzustand aus dem zweiten Logikzustand, wobei der dritte Übergang zu einem dritten Zeitpunkt nach dem zweiten Zeitpunkt und innerhalb der ersten Zeitspanne auftritt; und Erfassen eines vierten Übergangs des Signals in den zweiten Logikzustand aus dem ersten Logikzustand, wobei der vierte Übergang zu einem vierten Zeitpunkt nach dem dritten Zeitpunkt und auch innerhalb der ersten Zeitspanne auftritt, wobei die kumulative Zeitspanne eine zweite Zeitmenge einschließt, die zwischen dem dritten Zeitpunkt und dem vierten Zeitpunkt verstrichen ist, jedoch eine Zeitmenge ausschließt, die zwischen dem zweiten Zeitpunkt und dem dritten Zeitpunkt verstrichen ist; und/oder
das Erfassen des zweiten Übergangs ein Protokollieren des zweiten Zeitpunkts, zu dem der zweite Übergang auftrat, unter Verwendung eines ersten Interrupt-Handler umfasst.

4. Verfahren nach einem der Ansprüche 1 bis 3, wobei das Erfassen des ersten Übergangs das Protokollieren des ersten Zeitpunkts, zu dem der erste Übergang auftrat, unter Verwendung eines zweiten Interrupt-Handler umfasst.

5. Verfahren nach einem der Ansprüche 1 bis 4, wobei das Bestimmen, dass der Sensor (86, 1202) der Medikamentenabgabevorrichtung (10) zwischen den Zuständen gewechselt hat, ferner umfasst:
Bestimmen, ob eine Anzahl von Signalübergängen, die innerhalb der ersten Zeitspanne auftreten, einen Schwellenwert überschreitet; und
Bestimmen, dass der Sensor der Medikamentenabgabevorrichtung nur dann von dem außer Eingriff stehenden Zustand in den in Eingriff stehenden Zustand übergegangen ist, wenn die Anzahl vom Signalübergängen den Schwellenwert nicht überschreitet.

6. Verfahren nach einem der Ansprüche 1 bis 5, ferner umfassend:
Erfassen eines fünften Übergangs des Signals in den zweiten Logikzustand aus dem ersten Logikzustand, wobei der fünfte Übergang zu einem fünften Zeitpunkt nach der ersten Zeitspanne auftritt.

7. Verfahren nach Anspruch 6, ferner umfassend:
Bestimmen, ob sich, innerhalb einer zweiten Zeitspanne, die zu dem fünften Zeitpunkt beginnt, das Signal für eine zweite kumulative Zeitspanne, die gleich einer zweiten Schwellenwertdauer ist oder diese überschreitet, in dem zweiten Logikzustand befindet; und
Bestimmen, dass der Sensor (86, 1202) der Medikamentenabgabevorrichtung (10) zu dem fünften Zeitpunkt von dem in Eingriff stehenden Zustand in den außer Eingriff stehenden Zustand übergegangen ist, wenn die zweite kumulative Zeitspanne gleich der zweiten Schwellenwertdauer ist oder diese überschreitet, wobei, optional:
das Verfahren ferner das Erfassen eines sechsten Übergangs des Signals in den ersten Logikzustand aus dem zweiten Logikzustand umfasst, wobei der sechste Übergang zu einem sechsten Zeitpunkt nach dem fünften Zeitpunkt und innerhalb der zweiten Zeitspanne auftritt, wobei die zweite kumulative Zeitspanne eine Zeitmenge einschließt, die zwischen dem fünften Zeitpunkt und dem sechsten Zeitpunkt verstrichen ist; und/oder
eine erste Dauer der ersten Zeitspanne sich von einer zweiten Dauer der zweiten Zeitspanne unterscheidet; oder die erste Schwellenwertdauer sich von der zweiten Schwellenwertdauer unterscheidet; oder beides.

8. Verfahren nach einem der Ansprüche 1 bis 7, ferner umfassend:
Erfassen eines siebten Übergangs in den ersten Logikzustand aus dem zweiten Logikzustand, wobei der siebte Übergang zu einem siebten Zeitpunkt auftritt;
Bestimmen, ob sich, innerhalb einer dritten Zeitspanne, die zu dem siebten Zeitpunkt beginnt, das Signal für eine dritte kumulative Zeitspanne, die gleich der ersten Schwellenwertdauer ist oder diese überschreitet, in dem ersten Logikzustand befindet; und
Bestimmen, dass der Sensor (86, 1202) der Medikamentenabgabevorrichtung (10) zu dem siebten Zeitpunkt von dem außer Eingriff stehenden Zustand in den in Eingriff stehenden Zustand übergegangen ist, wenn die dritte kumulative Zeitspanne gleich der ersten Schwellenwertdauer ist oder diese überschreitet.

9. Verfahren nach einem der Ansprüche 1 bis 8, ferner umfassend:
Bestimmen einer Anzahl von Sensorübergängen, die innerhalb einer vierten Zeitspanne auftreten, die zu dem ersten Zeitpunkt beginnt, wobei die Anzahl von Sensorübergängen eine Medikamentenmenge anzeigt, die unter Verwendung der Medikamentenabgabevorrichtung (10) abgegeben wird.

10. Verfahren nach einem der Ansprüche 1 bis 9, wobei:
der erste Logikzustand einen angeblichen Zustand umfasst; und der zweite Logikzustand einen nicht angeblichen Zustand umfasst.

11. Nichtflüchtige, computerlesbare Speicherungsmedien, umfassend Anweisungen, die, wenn sie durch einen oder mehrere Prozessoren auf einer Rechenvorrichtung ausgeführt werden, betriebsfähig sind, um zu veranlassen, dass der eine oder die mehreren Prozessoren das Verfahren nach einem der Ansprüche 1 bis 10 ausführen.

12. Medikamentenabgabevorrichtung (10), umfassend:
ein Gehäuse (12), umfassend ein Reservoir, das ausreichend dimensioniert ist, um ein Medikament aufzunehmen;
eine Leiterplatte (77, 1200, 1400);
einen Sensor (86, 1202), der an der Leiterplatte montiert und betriebsfähig ist, um ein Signal auszugeben, wobei der Sensor betriebsfähig ist, um zwischen einem in Eingriff stehenden Zustand, in dem sich das Signal, das von dem Sensor ausgegeben wird, in einem ersten Logikzustand befindet, und einem außer Eingriff stehenden Zustand, in dem sich das Signal, das von dem Sensor ausgegeben wird, in einem zweiten Logikzustand befindet, zu wechseln; und
einen Mikrocontroller (1204, 1410) in elektrischer Kommunikation mit dem Sensor über einen Logikeingang in den Mikrocontroller, wobei der Mikrocontroller konfiguriert ist, um das Signal zu empfangen, das von dem Sensor ausgegeben wird,
**dadurch gekennzeichnet, dass** der Mikrocontroller ferner konfiguriert ist, um, basierend auf dem empfangenen Signal, zu bestimmen, ob der Sensor zwischen dem außer Eingriff stehenden Zustand und dem in Eingriff stehenden Zustand gewechselt hat, mindestens teilweise durch:
Bestimmen, ob sich, innerhalb einer ersten Zeitspanne, die an einem ersten Zeitpunkt beginnt, der einem ersten Übergang des Signals in den ersten Logikzustand aus dem zweiten Logikzustand entspricht, das Signal für eine kumulative Zeitspanne, die einer ersten Schwellenwertdauer gleicht oder diese überschreitet, in dem ersten Logikzustand befindet; und
Bestimmen, dass der Sensor von dem außer Eingriff stehenden Zustand in den in Eingriff stehenden Zustand übergegangen ist, wenn die kumulative Zeitspanne gleich der ersten Schwellenwertdauer ist oder diese überschreitet.

13. Medikamentenabgabevorrichtung (10) nach Anspruch 12, wobei der Mikrocontroller (1204, 1410) konfiguriert ist, um zu bestimmen, dass der Sensor (86, 1202) nicht von dem außer Eingriff stehenden Zustand in den in Eingriff stehenden Zustand übergegangen ist, wenn die kumulative Zeitspanne nicht gleich der ersten Schwellenwertdauer ist oder diese überschreitet.

14. Medikamentenabgabevorrichtung (10) nach einem der Ansprüche 12 bis 13, ferner umfassend ein drehbares Element (38), das relativ zu der Leiterplatte (77, 1200, 1400) drehbar ist, wobei das drehbare Element eine Reihe von Vorsprüngen (102) aufweist, die voneinander beabstandet sind, wobei das drehbare Element positioniert ist, um den Vorsprüngen zu ermöglichen, gegen den Sensor (86, 1202) zu gleiten, um den Sensor zwischen dem in Eingriff stehenden Zustand und dem außer Eingriff stehenden Zustand zu bewegen, während sich das drehbare Element dreht.

15. Medikamentenabgabevorrichtung (10) nach einem der Ansprüche 12 bis 14, ferner umfassend eine Widerstands-Kondensator-Schaltung (RC-Schaltung) (1424), die mit dem Sensor (86, 1202) und dem Mikrocontroller (1204, 1410) elektrisch gekoppelt ist, wobei das Signal ein gefiltertes Signal ist, und wobei die RC-Schaltung konfiguriert ist zum:
Empfangen eines ungefilterten Signals von dem Sensor; und
Übertragen des gefilterten Signals an den Mikrocontroller.

16. Medikamentenabgabevorrichtung (10) nach einem der Ansprüche 12 bis 14, ferner umfassend einen Zeitgeber (1704), der konfiguriert ist, um von einem anfänglichen Zeitpunkt aufwärts zu zählen, wobei, optional, das Bestimmen, ob der Sensor (86, 1202) zwischen dem außer Eingriff stehenden Zustand und dem in Eingriff stehenden Zustand übergegangen ist, ferner umfasst:
Erfassen des ersten Übergangs des Signals; und Bestimmen, unter Verwendung des Zeitgebers, des ersten Zeitpunkts, zu dem der erste Übergang auftrat; und/oder
Erfassen eines zweiten Übergangs des Signals in den zweiten Logikzustand aus dem ersten Logikzustand, wobei der zweite Übergang zu einem zweiten Zeitpunkt nach dem ersten Zeitpunkt und innerhalb der ersten Zeitspanne erfolgt; Bestimmen, unter Verwendung des Zeitgebers, des zweiten Zeitpunkts, zu dem der zweite Übergang auftrat; und Bestimmen einer Zeitmenge, die zwischen dem ersten Zeitpunkt und dem zweiten Zeitpunkt verstrichen ist.

17. Medikamentenabgabevorrichtung nach einem der Ansprüche 12 bis 16, ferner umfassend ein Medikament, das innerhalb des Reservoirs gehalten wird, optional wobei das Medikament Insulin ist.

## Revendications

1. Procédé de filtrage d'un signal émis à partir d'un capteur (86, 1202) d'un dispositif de délivrance de médicament (10), dans lequel le capteur est opérationnel pour effectuer une transition entre un état en prise où le signal émis à partir du capteur est dans un premier état logique et un état désolidarisé où le signal émis à partir du capteur est dans un second état logique, le procédé comprenant :
la détection d'une première transition du signal vers le premier état logique à partir du second état logique, la première transition se produisant à un premier point temporel, et étant **caractérisé en ce qu'**il comprend en outre :
la détermination de si, au sein d'une première période temporelle commençant au premier point temporel, le signal est dans le premier état logique pendant une période temporelle cumulée qui est égale à une première durée seuil ou la dépasse ; et
la détermination du fait que le capteur du dispositif de délivrance de médicament a effectué une transition à partir dudit état désolidarisé vers ledit état en prise au premier point temporel lorsque la période temporelle cumulée est égale à la première durée seuil ou la dépasse.

2. Procédé selon la revendication 1, comprenant en outre la détermination du fait que le capteur (86, 1202) du dispositif de délivrance de médicament (10) n'a pas effectué de transition à partir dudit état désolidarisé vers ledit état en prise lorsque la période temporelle cumulée n'est pas égale à la première durée seuil ou ne la dépasse pas.

3. Procédé selon l'une quelconque des revendications 1 à 2,
comprenant en outre la détection d'une deuxième transition du signal vers le second état logique à partir du premier état logique, la deuxième transition se produisant à un deuxième point temporel après le premier point temporel et au sein de la première période temporelle,
dans lequel la période temporelle cumulée comporte une première quantité de temps écoulée entre le premier point temporel et le deuxième point temporel, dans lequel, facultativement :
le procédé comprend en outre : la détection d'une troisième transition du signal vers le premier état logique à partir du second état logique, la troisième transition se produisant à un troisième point temporel après le deuxième point temporel et au sein de la première période temporelle ; et la détection d'une quatrième transition du signal vers le second état logique à partir du premier état logique, la quatrième transition se produisant à un quatrième point temporel après le troisième point temporel et également au sein de la première période temporelle, dans lequel la période temporelle cumulée comporte une seconde quantité de temps écoulée entre le troisième point temporel et le quatrième point temporel mais exclut une quantité de temps écoulée entre le deuxième point temporel et le troisième point temporel ; et/ou
la détection de la deuxième transition comprend l'enregistrement du deuxième point temporel auquel la deuxième transition s'est produite à l'aide d'un premier gestionnaire d'interruption.

4. Procédé selon l'une quelconque des revendications 1 à 3, dans lequel la détection de la première transition comprend l'enregistrement du premier point temporel auquel la première transition s'est produite à l'aide d'un second gestionnaire d'interruption.

5. Procédé selon l'une quelconque des revendications 1 à 4, dans lequel la détermination du fait que le capteur (86, 1202) du dispositif de délivrance de médicament (10) a effectué une transition entre lesdits états comprend en outre :
la détermination de si un nombre de transitions de signal se produisant au sein de la première période temporelle dépasse un seuil ; et
la détermination du fait que le capteur du dispositif de délivrance de médicament a effectué une transition à partir dudit état désolidarisé vers ledit état en prise uniquement lorsque le nombre de transitions de signal ne dépasse pas le seuil.

6. Procédé selon l'une quelconque des revendications 1 à 5, comprenant en outre :
la détection d'une cinquième transition du signal vers le second état logique à partir du premier état logique, la cinquième transition se produisant à un cinquième point temporel après la première période temporelle.

7. Procédé selon la revendication 6, comprenant en outre :
la détermination de si, au sein d'une deuxième période temporelle commençant au cinquième point temporel, le signal est dans le second état logique pendant une deuxième période temporelle cumulée qui est égale à une seconde durée seuil ou la dépasse ; et
la détermination du fait que le capteur (86, 1202) du dispositif de délivrance de médicament (10) a effectué une transition à partir dudit état en prise vers ledit état désolidarisé au cinquième point temporel lorsque la deuxième période temporelle cumulée est égale à la seconde durée seuil ou la dépasse, dans lequel, éventuellement :
le procédé comprend en outre la détection d'une sixième transition du signal vers le premier état logique à partir du second état logique, la sixième transition se produisant à un sixième point temporel après le cinquième point temporel et au sein de la deuxième période temporelle, dans lequel la deuxième période temporelle cumulée comporte une quantité de temps écoulée entre le cinquième point temporel et le sixième point temporel ; et/ou
une première durée de la première période temporelle est différente d'une seconde durée de la deuxième période temporelle ; ou la première durée seuil est différente de la seconde durée seuil ; ou les deux.

8. Procédé selon l'une quelconque des revendications 1 à 7, comprenant en outre :
la détection d'une septième transition vers le premier état logique à partir du second état logique, la septième transition se produisant à un septième point temporel ;
la détermination de si, au sein d'une troisième période temporelle commençant au septième point temporel, le signal est dans le premier état logique pendant une troisième période temporelle cumulée qui est égale à la première durée seuil ou la dépasse ; et
la détermination du fait que le capteur (86, 1202) du dispositif de délivrance de médicament (10) a effectué une transition à partir dudit état désolidarisé vers ledit état en prise au septième point temporel lorsque la troisième période temporelle cumulée est égale à la première durée seuil ou la dépasse.

9. Procédé selon l'une quelconque des revendications 1 à 8, comprenant en outre :
la détermination d'un nombre de transitions de capteur se produisant au sein d'une quatrième période temporelle commençant au quatrième point temporel, dans lequel le nombre de transitions de capteur indique une quantité de médicament délivrée à l'aide du dispositif de délivrance de médicament (10).

10. Procédé selon l'une quelconque des revendications 1 à 9, dans lequel :
le premier état logique comprend un état affirmé ; et le second état logique comprend un état désaffirmé.

11. Support de stockage non transitoire lisible par ordinateur comprenant des instructions qui, lorsqu'elles sont exécutées par un ou plusieurs processeurs sur un dispositif informatique, sont opérationnelles pour amener le ou les processeurs à exécuter le procédé selon l'une quelconque des revendications 1 à 10.

12. Dispositif de délivrance de médicament (10) comprenant :
un logement (12) comprenant un réservoir dimensionné de manière suffisante pour conserver un médicament ;
une carte à circuit imprimé (77, 1200, 1400) ;
un capteur (86, 1202) monté sur la carte à circuit imprimé et opérationnel pour émettre un signal, dans lequel le capteur est opérationnel pour effectuer une transition entre un état en prise où le signal émis à partir du capteur est dans un premier état logique et un état désolidarisé où le signal émis à partir du capteur est dans un second état logique ; et
un microcontrôleur (1204, 1410) en communication électrique avec le capteur à travers une entrée logique vers le microcontrôleur, dans lequel le microcontrôleur est configuré pour recevoir le signal émis à partir du capteur,
**caractérisé en ce que** le microcontrôleur est en outre configuré pour déterminer, en fonction du signal reçu, si le capteur a effectué une transition entre ledit état désolidarisé et ledit état en prise au moins en partie par :
la détermination de si, au sein d'une première période temporelle commençant à un premier point temporel correspondant à une première transition du signal vers le premier état logique à partir du second état logique, le signal est dans le premier état logique pendant une période temporelle cumulée qui est égale à une première durée seuil ou la dépasse ; et
la détermination du fait que le capteur a effectué une transition à partir dudit état désolidarisé vers ledit état en prise lorsque la période temporelle cumulée est égale à la première durée seuil ou la dépasse.

13. Dispositif de délivrance de médicament (10) selon la revendication 12, dans lequel le microcontrôleur (1204, 1410) est configuré pour déterminer que le capteur (86, 1202) n'a pas effectué de transition à partir dudit état désolidarisé vers ledit état en prise lorsque la période temporelle cumulée n'est pas égale à la première durée seuil ou ne la dépasse pas.

14. Dispositif de délivrance de médicament (10) selon l'une quelconque des revendications 12 à 13, comprenant en outre un élément rotatif (38) qui est rotatif par rapport à la carte à circuit imprimé (77, 1200, 1400), l'élément rotatif ayant une série de parties saillantes (102) qui sont espacées les unes des autres, l'élément rotatif étant positionné pour permettre aux parties saillantes de coulisser contre le capteur (86, 1202) pour déplacer le capteur entre ledit état en prise et ledit état désolidarisé à mesure que l'élément rotatif effectue une rotation.

15. Dispositif de délivrance de médicament (10) selon l'une quelconque des revendications 12 à 14, comprenant en outre un circuit de résistance-condensateur (RC) (1424) couplé électriquement au capteur (86, 1202) et au microcontrôleur (1204, 1410), dans lequel le signal est un signal filtré, et dans lequel le circuit RC est configuré pour :
recevoir un signal non filtré à partir du capteur ; et
transmettre le signal filtré au microcontrôleur.

16. Dispositif de délivrance de médicament (10) selon l'une quelconque des revendications 12 à 14, comprenant en outre un chronomètre (1704) configuré pour compter à partir d'un point temporel initial, dans lequel, éventuellement, la détermination de si le capteur (86, 1202) a effectué une transition entre ledit état désolidarisé et ledit état en prise comprend en outre :
la détection de la première transition du signal ; et la détermination, à l'aide du chronomètre, du premier point temporel auquel la première transition s'est produite ; et/ou
la détection d'une deuxième transition du signal vers le second état logique à partir du premier état logique, la deuxième transition se produisant à un deuxième point temporel après le premier point temporel et au sein de la première période temporelle ; la détermination, à l'aide du chronomètre, du deuxième point temporel auquel la deuxième transition s'est produite ; et la détermination d'une quantité de temps écoulée entre le premier point temporel et le deuxième point temporel.

17. Dispositif de délivrance de médicament selon l'une quelconque des revendications 12 à 16, comprenant en outre un médicament conservé au sein du réservoir, éventuellement dans lequel le médicament est de l'insuline.
